# EUROPEAN PATENT APPLICATION

(11) **EP 2 778 173 A1**
(43) Date of publication of application: **17.09.2014**
(21) Application number: 13305309.0
(22) Date of filing: 15.03.2013
(51) Int. Cl.: C07K 16/12

(54) **Antibody directed against anthrax toxins and its uses**

(71) Applicant: Laboratoire Français du Fractionnement et des Biotechnologies, 91940 Les Ulis (FR)
(72) Inventor: The designation of the inventor has not yet been filed
(74) Representative: Jupin, Claude Christian Marie

(57) **Abstract**

The present invention relates to antibodies which are directed against anthrax toxins, and especially the LF and EF toxins. This invention also pertains to the medical uses of these anti-anthrax toxins antibodies.

## Description

### FIELD OF THE INVENTION

The present invention relates to the field of medical treatments against anthrax toxins.

### BACKGROUND OF THE INVENTION

In 2001, the intentional release of anthrax spores through the U.S. postal system confirmed that *Bacillus anthracis* can cause high morbidity and mortality, despite the use of powerful antibiotherapy and resuscitation techniques. The pathogenesis of *B. anthracis* is largely due to a tripartite protein complex consisting of a component binding cellular receptors, the protective antigen (PA), and two catalytic components, the lethal factor (LF) and the edema factor (EF). PA and LF combine to form the lethal toxin (LT), and PA and EF combine to form the edema toxin (ET). However, only LT is recognized as being essential for anthrax pathogenesis (for a review, see [Mock M, Fouet A (2001) anthrax. Annu Rev Microbiol 55: 647-671 ; Moayeri M, Leppla SH (2009) Cellular and systemic effects of anthrax lethal toxin and edema toxin. Mol Aspects Med 30: 439-455]). EF and LF bind to PA with high affinities (KD = 1nM) [Elliott JL, Mogridge J, Collier RJ (2000) A quantitative study of the interactions of Bacillus anthracis edema factor and lethal factor with activated protective antigen. Biochemistry 39: 6706-6713.]; their binding is competitive and involves their N-terminal domains, which present a conserved structure [Pannifer AD, Wong TY, Schwarzenbacher R, Renatus M, Petosa C, et al. (2001) Crystal structure of the anthrax lethal factor. Nature 414: 229-233; Petosa C, Collier RJ, Klimpel KR, Leppla SH, Liddington RC (1997) Crystal structure of the anthrax toxin protective antigen. Nature 385: 833-838; Lacy DB, Mourez M, Fouassier A, Collier RJ (2002) Mapping the anthrax protective antigen binding site on the lethal and edema factors. J Biol Chem 277: 3006-3010].

For antibiotic treatments of anthrax to be effective, they must be administered rapidly after infection [Burnett JC, Henchal EA, Schmaljohn AL, Bavari S (2005) The evolving field of biodefence: therapeutic developments and diagnostics. Nat Rev Drug Discov 4: 281-297] as lethal amounts of anthrax toxins are quickly secreted into the bloodstream. Antibiotic efficacy is also limited by the existence of *B. anthracis* antibioresistance [Patra G, Vaissaire J, Weber-Levy M, Le Doujet C, Mock M (1998) Molecular characterization of Bacillus strains involved in outbreaks of anthrax in France in 1997. J Clin Microbiol 36: 3412-3414; Lightfoot N, Scott R, Turnbull B (1990) Antimicrobial susceptibility of Bacillus anthracis. Salisbury Med Bull Suppl 68; Durmaz R, Doganay M, Sahin M, Percin D, Karahocagil MK, et al. (2012) Molecular epidemiology of the Bacillus anthracis isolates collected throughout Turkey from 1983 to 2011. Eur J Clin Microbiol Infect Dis 31: 2783-2790]. However, it was demonstrated in animal models of anthrax that the passive transfer of neutralizing antibodies directed against either PA or LF can improve the outcome of the disease [Little SF, Ivins BE, Fellows PF, Friedlander AM (1997) Passive protection by polyclonal antibodies against Bacillus anthracis infection in guinea pigs. Infect Immun 65: 5171-5175]. Consequently, considerable efforts have been devoted, since 2001, to the development of recombinant antibodies to be used to complement antibiotic therapy (for a review, see [Froude JW, 2nd, Thullier P, Pelat T (2011) Antibodies against anthrax: mechanisms of action and clinical applications. Toxins (Basel) 3: 1433-1452; Chen Z, Moayeri M, Purcell R (2011) Monoclonal Antibody Therapies against anthrax. Toxins 3: 1004-1019]), and they resulted in the recent FDA approval of raxibacumab for the treatment of inhalational anthrax [Fox JL (2013) anthrax drug first antibacterial mAb to win approval. Nat Biotechnol 31: 8]. However, concerns have been raised about the use of anti-PA antibodies alone [Baillie LW (2006) Past, imminent and future human medical countermeasures for anthrax. J Appl Microbiol 101: 594-606], because it was feared that PA could be naturally or voluntarily modified so as to escape binding by anti-PA antibodies while retaining its biological activity [Rosovitz MJ, Schuck P, Varughese M, Chopra AP, Mehra V, et al. (2003) J Biol Chem 278: 30936-30944]. Consequently anti-LF antibodies have also been considered for anthrax therapy [Baillie LW (2006) Past, imminent and future human medical countermeasures for anthrax. J Appl Microbiol 101: 594-606.]. Another possible advantage of such antibodies is that they could potentially synergize with anti-PA antibodies [Brossier F, Levy M, Landier A, Lafaye P, Mock M (2004) Functional analysis of Bacillus anthracis protective antigen by using neutralizing monoclonal antibodies. Infect Immun 72: 6313-6317 ; Albrecht MT, Li H, Williamson ED, LeButt CS, Flick-Smith HC, et al. (2007) Human monoclonal antibodies against anthrax lethal factor and protective antigen act independently to protect against Bacillus anthracis infection and enhance endogenous immunity to anthrax. Infect Immun 75: 5425-5433].

The first recombinant anti-LF antibody fragment, scFv 2LF, was isolated using an original strategy, based on the construction of phage-displayed libraries from immunized macaques (*Macaca fascicularis*)*.* Macaque and human antibodies are similar and, if needed for therapeutic purposes, the immunogenicity of macaque antibodies can be decreased by germline humanization [Pelat T, Thullier P (2009) Non-human primate immune libraries combined with germline humanization: an (almost) new, and powerful approach for the isolation of therapeutic antibodies. Mabs 1: 377-381; Pelat T, Bedouelle H, Rees AR, Crennell SJ, Lefranc MP, et al. (2008) Germline humanization of a non-human primate antibody that neutralizes the anthrax toxin, by in vitro and in silico engineering. J Mol Biol 384: 1400-1407]. The neutralization of the lethal toxin by scFv 2LF is due to the inhibition of the interaction between PA and LF, i.e. by the inhibition of the formation of the toxin [18]. This mechanism is shared by other LF-neutralizing antibodies [Little SF, Leppla SH, Friedlander AM (1990) Production and characterization of monoclonal antibodies against the lethal factor component of Bacillus anthracis lethal toxin. Infect Immun 58: 1606-1613; Zhao P, Liang X, Kalbfleisch J, Koo HM, Cao B (2003) Neutralizing monoclonal antibody against anthrax lethal factor inhibits intoxication in a mouse model. Hum Antibodies 12: 129-135; Lim NK, Kim JH, Oh MS, Lee S, Kim SY, et al. (2005) An anthrax lethal factorneutralizing monoclonal antibody protects rats before and after challenge with anthrax toxin. Infect Immun 73: 6547-6551] but their epitopes remain to be accurately mapped.

The development of novel antibodies for neutralizing anthrax toxin is thus of general interest for the prevention and effective treatment of anthrax.

### SUMMARY OF THE INVENTION

The present invention relates to a recombinant antibody comprising :
(i) a variable region binding to an epitope of the LF anthrax toxin [of SEQ ID N° X1] comprising the amino acid residues H229, R230, Q234, L235 and Y236 thereof, and
(ii) a constant region of a human immunoglobulin of the gamma isotype

In some embodiments, of the said recombinant antibody, the variable region comprises :
- a VL region comprising an amino acid sequence having at least 90% amino acid identity with the amino acid sequence of SEQ ID N° 6, and
- a VH region comprising an amino acid sequence having at least 90% amino acid identity with the amino acid sequence of SEQ ID N° 9.

In some embodiments, of the said recombinant antibody, the variable region comprises :
- a CL region comprising an amino acid sequence having at least 90% amino acid identity with the amino acid sequence of SEQ ID N° 7, and
- a CH region comprising an amino acid sequence having at least 90% amino acid identity with the amino acid sequence of SEQ ID N° 10.

This invention also relates to a nucleic acid encoding a recombinant antibody as described above.

In some embodiments, the said nucleic acid comprises the following nucleic acid sequences:
- a nucleic acid encoding the VL region, comprising a nucleic acid sequence having at least 90% nucleotide identity with SEQ ID N° 12,
- a nucleic acid encoding the VH region, comprising a nucleic acid sequence having at least 90% nucleotide identity with SEQ ID N° 15,
- a nucleic acid encoding the CL region, comprising a nucleic acid sequence having at least 90% nucleotide identity with SEQ ID N° 13,
- a nucleic acid encoding the CH region, comprising a nucleic acid sequence having at least 90% nucleotide identity with SEQ ID N° 16,

This invention also pertains to a vector, preferably an expression vector, comprising a nucleic acid as described above.

The invention also concerns a host cell comprising a nucleic acid or with a vector, preferably an expression vector, as described above, which encompasses a host cell that has been transfected with a vector, preferably an expression vector, as described above.

This invention also pertains to a pharmaceutical composition comprising a recombinant antibody as described above, in combination with one or more pharmaceutically acceptable excipients.

This invention also concerns the use of an antibody binding both to :
a) an epitope of the LF anthrax toxin comprising the amino acid residues H229, R230, Q234, L235 and Y236, and
b) an epitope of the EF anthrax toxin comprising the amino acid residues H253, R254, E258, L259 and Y260 thereof,
for preventing or treating a disorder or a disease caused by the presence of an anthrax toxin in an individual.

In some embodiments of the said medical use, the said antibody is an antibody as described above.

The present invention also relates to a method for preventing or treating a disorder or a disease caused by the presence of an anthrax toxin in an individual, comprising a step of administering to an individual in need thereof a recombinant antibody as described above.

This invention also pertains to a nucleic acid encoding a recombinant chimeric antibody as described above.

This invention also concerns a method for the screening of compounds that neutralize anthrax toxins, comprising the steps of :
a) bringing into contact a candidate compound with an LF anthrax toxin or a fragment thereof comprising the epitope comprising the amino acid residues H229, R230, Q234, L235 and Y236 thereof,
b) determining the occurrence of a binding event between the said candidate compound and the said LF anthrax toxin of the said fragment thereof,
c) selecting the said candidate compound if a binding event between the said candidate compound and the said LF anthrax toxin of the said fragment thereof is determined at step b).

In some embodiments, the method above further comprises the following steps :
d) bringing into contact a candidate compound with an EF anthrax toxin or a fragment thereof comprising the comprising the amino acid residues H253, R254, E258, L259 and Y260 thereof,
e) determining the occurrence of a binding event between the said candidate compound and the said EF anthrax toxin of the said fragment thereof,
f) selecting the said candidate compound if a binding event between the said candidate compound and the said EF anthrax toxin of the said fragment thereof is determined at step e).
and wherein the order of (i) steps a)-c) and (ii) steps d)-e), respectively, is indifferent.

In certain embodiments of the screening method above, the candidate compound consists of an antibody or an antibody fragment comprising the variable region thereof.

### DESCRIPTION OF THE FIGURES

**Figure 1****.** Evaluation of the reactivity of scFv 2LF with EF by ELISA and western blot.
   Reactivity of scFv 2LF with EF in ELISA. Reactivity of scFv 2LF with EF (curve n° 1), and LF (curve n° 2) and KLH (curve n° 3) were used as positive and negative controls, respectively. ScFv 2LF reacted with EF, though less strongly than with LF at high scFv concentrations.
**Figure 2****.** Biacore experiment to measure the affinity of scFv 2LF for EF.
   Sensorgrams were obtained at EF concentrations of 220 nM (curve n° 1), 110 nM (curve n° 2), 55 nM (curve n° 3), 28 nM curve n° 4) and 14 nM (curve n° 5) on a CM5 chip sensitized by 250 response units of scFv 2LF. The affinity of scFv 2LF for EF was measured as 5 nM.
**Figure 3****.** IgG 2LF inhibits the formation of edema induced by the edema toxin (ET).
   Evolution of the sizes (dorsal/plantar) of mouse footpads after injection of 20 µg ET (blue), or of 20 µg ET pre-mixed with 2 µg (red) or 5 µg (yellow) or 10µg (light blue) of IgG 2LF. Injection of PBS only (purple) was used as a negative control. Curves represent triplicate measurements for each timepoint. Pre-mixing with 10 µg of IgG 2LF completely abolished the formation of edema by 20 µg ET, and pre-mixing with 5 µg of IgG 2LF limited the size of the edema and reduced its duration. Pre-mixing of 20 µg ET with 2 µg of IgG 2LF had no appreciable effect.

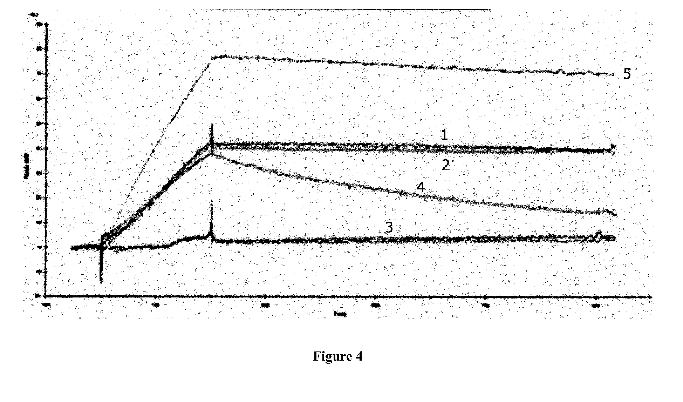

**Figure 4****.** Biacore experiment to evaluate the reactivity of the five LF variants in which each epitope candidate had been shaved to alanine, and localization of these epitope candidates on a view of the LFN surface.
   The Biacore experiment was performed on a CM5 chip sensitized by scFv 2LF. The curve corresponding to the LF variant in which LF(97-103) was shaved is in purple, the curves corresponding to the shaving of LF(136-143), LF(178-184), LF(227-231) and LF(231-236) are in green, blue, red and orange, respectively. The positive control (commercially available LF) curve is in gray. Only the reactivities of LF variants in which LF(178-184), LF(227-231), LF(231-236) had been shaved were significantly (three-fold or more) lower than the positive control value.

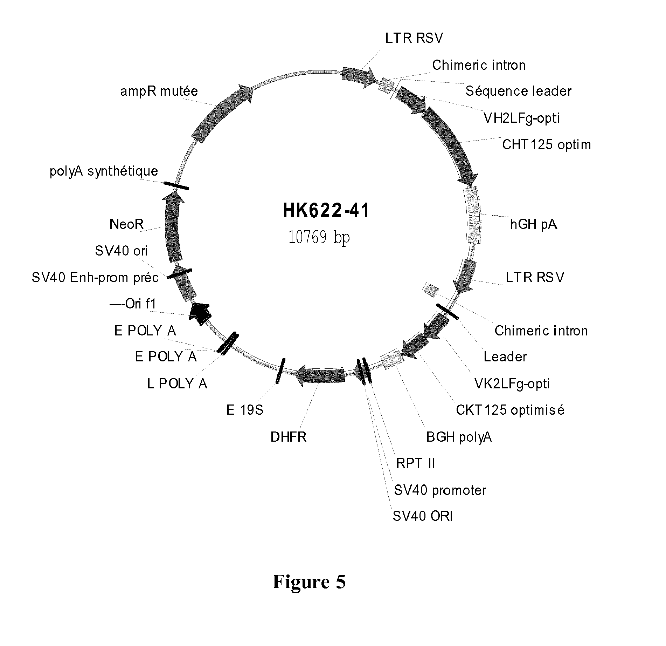

**Figure 5****.** Schematic representation of the map of the expression vector HK622-41 encoding the anti-LF/EF chimeric antibody.

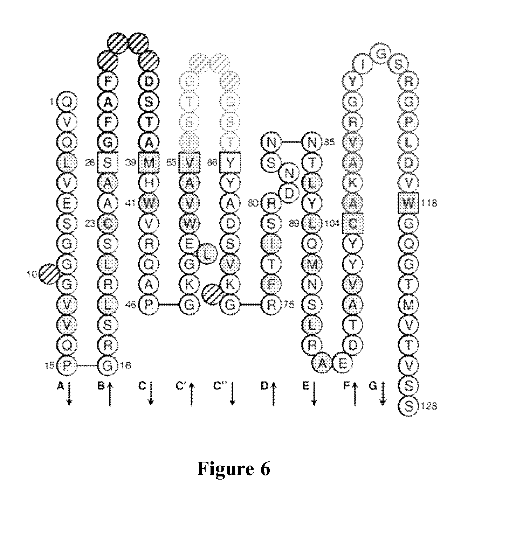

**Figure 6****.** Heavy chain IMGT "collier de perles" graphical 2-dimensional representation of the anti-LF/EF chimeric antibody.

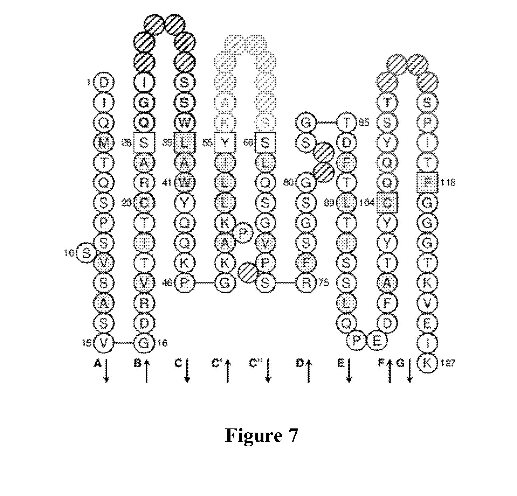

**Figure 7****.** Light chain IMGT "collier de perles" graphical 2-dimensional representation of the anti-LF/EF chimeric antibody.

### DETAILED DESCRIPTION OF THE INVENTION

The present invention provides for antibodies directed against the LF anthrax toxin which are especially useful for preventing or treating a disorder or a disease caused by the presence of an anthrax toxin in an individual.

As shown in the examples herein, the 2LF ScFv entity, that was initially described by Pelat et al. (2007, Antimicrobial Agents and Chemotherapy, Vol. 51(8) : 2758-2764) for its binding properties to the anthrax LF toxin, also efficiently binds to the anthrax EF toxin.

Because cross-reactivity of 2LF ScFv with both LF and EF has been determined according to the invention, an accurate mapping of the epitopes of scFv 2LF on both LF and EF was performed, by designing a specific and original mapping method involving four different steps. First, the cross-reactivity of scFv 2LF with EF was tested and the cross-neutralization of ET was characterized. Second, an *in silico* method was developed to identify regions exposed to the solvent and shared between LF and EF, as these regions were regarded as epitope candidates. In the third part, these epitope candidates were tested by mutating their residues to alanine, thereby mapping the epitope precisely. Lastly, the homolog of this epitope on EF was experimentally demonstrated to constitute the epitope ofscFv 2LF on EF.

Consequently, it has been shown according to the invention that the 2LF ScFv entity has the ability to alter both (i) the formation of complexes between the PA and the LF anthrax toxins for forming the lethal toxin and (ii) the formation of complexes between the PA and the EF toxins for forming the edema toxin.

In particular, it is shown in the examples herein that the 2LF ScFv entity is able to neutralize *in vivo* both LF and EF toxic effects.

For LF, it is preferably referred to the amino acid sequence of SEQ ID N°1 herein.

For EF, it is preferably referred to the amino acid sequence of SEQ ID N° 2 herein.

Further, by using a specifically designed epitope mapping method, it has been identified according to the present invention an epitope which is "common" to both the LF and the EF anthrax toxins, which epitope consists of a new and highly valuable target for selecting or designing anti-anthrax compounds of diagnosis or of therapeutic interest.

The epitopes which are identified herein and which are contained in the LF and the EF anthrax toxins are not strictly identical. These two epitopes contained in the LF and the EF anthrax toxins, respectively, comprise five essential amino acid residues, with four of the said five amino acid residues that are common to both epitopes, the fifth amino acid residue consisting of (i) a glutamine residue located at position 234 of the LF anthrax toxin of SEQ ID N° 1 and of (ii) a glutamic acid residue located at position 258 of the EF anthrax toxin of SEQ ID N° 2.

More precisely, the structurally close epitopes identified herein and that are disclosed for the first time in the present specification are the following :
- the epitope of the LF anthrax toxin comprising the amino acid residues H229, R230, Q234, L235 and Y236 thereof, and
- the epitope of the EF anthrax toxin comprising the amino acid residues H253, R254, E258, L259 and Y260 thereof.

Thus, the epitopes of interest that are contained in both the LF and EF anthrax toxins, respectively, comprise, or consist of, a peptide having the following amino acid sequences:
(i) LF epitope : NH2-HRDVLQLY-COOH (SEQ ID N° 3)
(ii) EF epitope : NH2-HRTVLELY-COOH (SEQ ID N° 4)

The common LF/EF epitope of interest that is identified according to the invention comprises, or consists of, the following amino acid sequence :
NH2-H-R-X1-V-L-X2-L-Y (SEQ ID N° 5), wherein :
   - X1 means an amino acid residue selected from the group comprising an aspartic acid (also termed Asp or D) and a Threonine residue (also termed Thr or T), and
   - X2 means an amino acid residue selected from the group comprising a Glutamine residue (also termed Gln or Q) and a Glutamic acid residue (also termed Glu or E).

For the purpose of the present invention, an epitope comprising, or consisting of, an amino acid sequence selected from the group comprising SEQ ID N°3, SEQ ID N° 4 or SEQ ID N° 5 may also be interchangeably termed the "neutralization epitope" or "anthrax neutralization epitope" or "anthrax toxin neutralization epitope" or "LF neutralization epitope" or "EF neutralization epitope" herein.

Altogether, the results obtained by the inventors have allowed to determine that powerful anti-anthrax substances may be found among those binding to an epitope comprised in the LF anthrax toxin that is also present in the EF anthrax toxin. Indeed, such powerful anti-anthrax substances include those which are suitable for their administration to human and animal bodies and which comprise at least the domains of the 2LF ScFv entity which are responsible for the binding properties of the said 2LF ScFv entity to the LF anthrax toxin, which powerful anti-anthrax substances include antibodies.

Further, it has been shown that a chimeric antibody according to the invention has at least the same affinity for EF than the 2LF ScFv.

Such anti-anthrax substances consisting of antibodies directed against the newly identified epitope of the LF anthrax toxin that is common to the EF anthrax toxin are provided by the present invention.

The present invention relates to a recombinant antibody comprising :
(i) a variable region binding to an epitope of the LF anthrax toxin comprising the amino acid residues H229, R230, Q234, L235 and Y236 thereof, and
(ii) a constant region of a human immunoglobulin of the gamma isotype

For the purpose of the present specification, a recombinant as defined above may also be termed "anti-LF/EF" antibody. anti-LF/EF antibody

As used herein an "antibody" is intended to mean an immunoglobulin molecule having the ability to specifically bind to a particular antigen. In embodiments, immunoglobulins of the gamma isotype (also termed IgG) are heterodimers consisting of 2 heavy chains and of 2 light chains, bound to each other through disulfide bridges. Each chain is composed, at the N-terminal position, of a variable region or domain (encoded by the rearranged genes V-J for the light chain and V-D-J for the heavy chain) specific for the antigen against which said immunoglobulin is directed, and at the C-terminal position, of a constant region, consisting of a single domain CL for the light chain or of 3 domains (CH1, CH2 and CH3) for the heavy chain. The association of the variable domains and of the CH1 and CL domains of the heavy and light chains form Fab portions, which are connected to the Fc region through a very flexible hinge region allowing each Fab to bind to its antigen target, while the Fc region, mediating the effector properties of the antibody, remains accessible to the immune effectors, phagocytes or killer cells, and the complement; these constant regions are not involved in the binding to the antigen. The Fc region, composed of the 2 globular domains, CH2 and CH3, is glycosylated on the CH2 domain with the presence, on each of both chains, of a biantennary N-glycan of the lactosamine type, bound to Asn 297. As it is known in the art, the variable region, it is involved in the binding of the antibody to the epitope thereof.

Embodiments of a recombinant antibody according to the invention (anti-LF/EF antibody) contain a variable region of non-human origin, and especially a variable region encoded by nucleic sequences originating from the genome of Cynomolgus macaque (*Macaca fascicularis*) and a constant region originating from the human genome (*Homo sapiens*)*.* These embodiments of a recombinant antibody according to the invention are preferably termed "chimeric antibody" in the present specification. However, it is recalled that the macaque germline V, (D), or J alleles are very similar to the human germline V, (D), or J alleles. For this reason, a reduced immune response against a recombinant antibody of the invention, or even no immune response against the said antibody is expected following its adminstration to human individuals in need thereof.

In certain embodiments of an antibody according to the invention, the variable region thereof comprises :
- a VL region comprising an amino acid sequence having at least 90% amino acid identity with the amino acid sequence of SEQ ID N° 6, and
- a VH region comprising an amino acid sequence having at least 90% amino acid identity with the amino acid sequence of SEQ ID N° 9.

According to the invention, a first nucleic acid having at least 90% identity with a second nucleic acid of reference, will have at least 90%, preferably at least 91%, 92%, 93%, 94%, 95%, 96%, 97%, 97.5%, 98%, 98.3% 98.6%, 99%, 99.6% nucleotide identity with the said second nucleic acid of reference.

According to the invention, a first polypeptide that has at least 90% identity with a second polypeptide of reference, will have at least 90%, preferably at least 91%, 92%, 93%, 94%, 95%, 96%, 97%, 97.5%, 98%, 98.3% 98.6%, 99%, 99.6% amino-acid identity with the said second polypeptide of reference.

As used herein, the "percentage of identity" between two nucleic acid sequences or between two polypeptide sequences is determined by comparing both optimally aligned sequences through a comparison window.

The portion of the nucleotide or amino-acid sequence in the comparison window may thus include additions or deletions (for example "gaps") as compared to the reference sequence (which does not include these additions or these deletions) so as to obtain an optimal alignment between both sequences.

The identity percentage is calculated by determining the number of positions at which an identical nucleic base, or an identical amino-acid residue, can be noted for both compared sequences, then by dividing the number of positions at which identity can be observed between both nucleic bases, or between both amino-acid residues, by the total number of positions in the comparison window, then by multiplying the result by hundred to obtain the percentage of nucleotide identity between the two sequences or the percentage of amino acid identity between the two sequences.

The comparison of the sequence optimal alignment may be effected by a computer using known algorithms.

Most preferably, the sequence identity percentage is determined using the CLUSTAL W software (version 1.82) the parameters being set as follows: (1) CPU MODE=ClustalW mp; (2) ALIGNMENT="full"; (3) OUTPUT FORMAT="aln w/numbers"; (4) OUTPUT ORDER="aligned"; (5) COLOR ALIGNMENT="no"; (6) KTUP (word size)="default"; (7) WINDOW LENGTH="default"; (8) SCORE TYPE="percent"; (9) TOPDIAG="default"; (10) PAIRGAP="default"; (11) PHYLOGENETIC TREE/TREE TYPE="none"; (12) MATRIX="default"; (13) GAP OPEN="default"; (14) END GAPS="default"; (15) GAP EXTENSION="default"; (16) GAP DISTANCES="default"; (17) TREE TYPE="cladogram" and (18) TREE GRAP DISTANCES="hide".

In the structure of a recombinant antibody according to the invention (anti-LF/EF antibody), the constant region thereof defines an antibody of the IgG isotype. As it is well known in the art, antibodies of the IgG isotype are those which are the most abundant in the blood circulation and in the extracellular fluids. The IgG immunoglobulins which consist of monomer antibodies allow the human and animal body to control infection of body tissues by pathogenic organisms and to neutralize deleterious substances produced by the said pathogenic organisms.

In certain embodiments of a recombinant antibody according to the invention, the constant region is selected in the group comprising gamma 1, gamma 2, gamma 3 or gamma 4 isotype.

In preferred embodiments of a recombinant antibody according to the invention, the constant region is selected in the group comprising gamma 1 and gamma 2 isotype and is most preferably of the gamma 1 isotype (IgG1 antibody).

In preferred embodiments of a recombinant antibody according to the invention the constant region consists of a constant region of a human IgG1 antibody and comprises :
- a CL region comprising an amino acid sequence having at least 90% amino acid identity with the amino acid sequence of SEQ ID N° 7, and
- a CH region comprising an amino acid sequence having at least 90% amino acid identity with the amino acid sequence of SEQ ID N° 10.

The recombinant antibodies according to the inventions, which include the chimeric antibodies, comprise individual heavy (H) and light (L) immunoglobulin chains. A chimeric H chain comprises an antigen binding region derived from the H chain of a non-human antibody specific for the LF/EF neutralization epitope described herein, which H chain-derived antigen binding region is linked to at least a portion of a human H chain constant region (CH region).

As used herein, the term "antigen binding region" refers to that portion of an antibody molecule which contains the amino acid residues that interact with an antigen and confer on the antibody its specificity and affinity for the antigen. The antibody region includes the "framework" amino acid residues necessary to maintain the proper conformation of the antigen-binding residues.

As used herein, the term "chimeric antibody" includes monovalent, divalent or polyvalent immunoglobulins. A monovalent chimeric antibody is a dimer (HL)) formed by a chimeric H chain associated through disulfide bridges with a chimeric L chain. A divalent chimeric antibody is tetramer (H24) formed by two HL dimers associated through at least one disulfide bridge. A polyvalent chimeric antibody can also be produced, for example, by employing a CR region that aggregates (e.g., from an IgM H chain, or y chain).

Most preferably, a recombinant chimeric antibody according to the invention consists of a monovalent antibody of the gamma isotype.

Antibodies, fragments or derivatives having chimeric H chains can be prepared by appropriate association of the individual polypeptide chains, as taught, for example by Sears p et al., (1975, Proc. Natl. Acad. Sci. USA 72:353-357). With this approach, hosts expressing chimeric H chains are separately cultured from hosts expressing L chains, and the immunoglobulin chains are separately recovered and then associated. Alternatively, the hosts can be co-cultured and the chains allowed to associate spontaneously in the culture medium, followed by recovery of the assembled immunoglobulin, fragment or derivative.

The antigen binding region of the chimeric antibody of the present invention is derived preferably from a non-human antibody specific for the LF/EF neutralizing epitope described herein. Preferred sources for the DNA encoding such a non-human antibody include cell lines which produce antibody, preferably hybrid cell lines commonly known as hybridomas.

In some embodiments, a chimeric EF/LF antibody according to the invention may be "germline humanized", for example by using the method described by Hwang et al. (2008, Methods, Vol. 36(1) : 35-42) or by using the method described by Pelat et al. (2008, J Mol Biol, Vol. 384(5) : 1400-1407). In some embodiments, a chimeric EF/LF antibody according to the invention may be "germline humanized" according to the method disclosed in the PCT application published under n° WO 2012/038609.

The hybrid cells may be formed by the fusion of a non-human anti-"LF/EF neutralizing" antibody-producing cell, typically a spleen cell of an animal immunized against either natural or recombinant LF or EF, and especially a spleen cell of a cynomolgus macaque immunized against either natural or recombinant LF or EF.

The antibody-producing cell contributing the nucleotide sequences encoding the antigen-binding region of the chimeric antibody of the present invention may also be produced by transformation of a non-human cell, such as a primate cell. For example, a B lymphocyte which produces an "LF/EF neutralizing" antibody may be infected and transformed with a virus such as Epstein-Barr virus to yield an immortal anti-TNF producing cell (Kozbor petal. Immunol. Today 4:72-79 (1983)). Alternatively, the B lymphocyte may be transformed by providing a transforming gene or transforming gene product, as is well-known in the art.

Preferably, the antigen binding region will be of macaque origin. In other embodiments, the antigen binding region may be derived from other animal species, in particular rodents such as mouse, rabbit, rat or hamster.

The second fusion partner, which provides the immortalizing function, may be lymphoblastoid cell or a plasmacytoma or myeloma cell, which is not itself an antibody producing cell, but is malignant. Illusrative fusion partner cells include the hybridoma SP2/0-Ag14, abbreviated as SP2/0 (ATCC CRL1581) and the myeloma P3X63Ag8 (ATCC TIB9), or its derivatives (see: Hartlow, E. et al., Antibodies: A Laboratory Manual, Cold Spring Harbor Laboratory Press, Cold Spring Harbor, NY, 1988)).

Murine hybridomas which produce a anti-LF/EF antibody specific for the LF/EF neutralizing epitope described herein may be formed by the fusion of a mouse fusion partner cell, such as SP2/0, and spleen cells from a Cynomolgus macaque immunized against purified or recombinant LF or EF anthrax toxin, or immunized against peptides thereof comprising the LF/EF epitope described herein. Such peptides include peptides comprising the amino acid residues 229-236 of the LF anthrax toxin and peptides comprising the amino acid residues 253-260 of the LF anthrax toxin.

The techniques to raise antibodies of the present invention, i.e. anti-LF/EF antibodies, to small peptide sequences that recognize and bind to those sequences in the free or conjugated form or when presented as a native sequence in the context of a large protein are well known in the art.

This invention also concerns a nucleic acid comprising nucleic acid sequences encoding a recombinant antibody as defined in the present specification.

This invention pertains to a nucleic acid comprising one or more of the following nucleic acid sequences :
- a nucleic acid encoding the VL region, comprising a nucleic acid sequence having at least 90% nucleotide identity with SEQ ID N° 12,
- a nucleic acid encoding the VH region, comprising a nucleic acid sequence having at least 90% nucleotide identity with SEQ ID N° 15,
- a nucleic acid encoding the CL region, comprising a nucleic acid sequence having at least 90% nucleotide identity with SEQ ID N° 13, and
- a nucleic acid encoding the CH region, comprising a nucleic acid sequence having at least 90% nucleotide identity with SEQ ID N° 16.

It is a further object of the present invention to provide a vector comprising a nucleic acid encoding an anti-LF/EF antibody of the invention.

In some embodiments, the said vector consists of an expression vector comprising the nucleic sequences encoding the light chain and the heavy chain of an anti-LE/EF antibody as described herein. In some embodiments, the said vector comprises the nucleic acid sequences encoding (i) the complete light chain and (ii) the complete heavy chain, respectively, the expression of each of these nucleic acid sequences being placed under the control of appropriate promoter sequences. In some embodiments the said vector comprises the nucleic acid sequences encoding (i) the variable region of the light chain, (ii) the constant region of the light chain, (iii) the variable region of the heavy chain and (iv) the constant region of the heavy chain, respectively, the expression of each of these nucleic acid sequences being placed under the control of appropriate promoter sequences. In some embodiments, the antibody-encoding sequences contained in the said vector encode the relevant antibody polypeptide that is fused to a leader peptide sequence. When expressed, the fusion polypeptide is matured in the relevant antibody polypeptide by enzyme cleavage of the leader peptide sequence contained therein, in the host cell in which the said vector is comprised.

In some embodiments, the said vector consists of the vector termed "HK622-41" that is illustrated in Figure 5.

As used herein, a "vector" refers to a nucleic acid wherein the sequence of interest may be inserted by restriction, then ligation for the transport to and within various genetical environments or for the expression in a host cell. Vectors are for example plasmids, cosmids, yeast artificial chromosomes (YAC), bacterial artificial chromosomes (BAC) and artificial chromosomes derived from the bacteriophage P1 (PAC), virus-derived vectors. A cloning vector is a vector capable of replicating in a host cell and which in addition is characterized by the presence of one or more endonuclease restriction sites. An expression vector is a vector wherein the DNA sequence of interest may be inserted through restriction or ligation techniques so as to enable its replication and/or transcription to RNA. The vectors may contain in addition one or more markers for selecting or identifying the cells that have been transformed or transfected with the vector.

These nucleic acids can be incorporated into a recombinant vector for the cloning or for the expression of the anti-LF/EF antibodies of the invention.

The present invention includes all the recombinant vectors containing coding sequences for the purpose of eukaryotic or prokaryotic cell transformation, transfection or gene therapy. Such vectors will be prepared according to conventional methods in molecular biology and will comprise in addition a suitable promoter, optionally a signal sequence for the export or secretion, and regulatory sequences required for the transcription of the nucleotide sequence.

In some embodiments, fusion polypeptide may be required for purifying the antibodies of the present invention. The fusion domain may include for example a polyhistidine tail enabling the purification onto Ni²⁺ columns or a filamentous phage membrane anchor, which is particularly useful for screening of a library, according to the technology called "phage display".

A vector to be suitably used in the context of the present invention is a recombinant DNA molecule adapted for receiving and expressing a first and a second DNA sequence, so as to enable the expression of heterodimer antibodies, such as a full-length antibody or F(ab')2 or Fab fragments according to the invention. Such a vector provides a system for independently cloning both DNA sequences in two separate cassettes that are present in the vector, so as to form two distinct cistrons for the expression of a first and a second polypeptide from the heterodimer antibody. Such an expression vector is called a dicistronic vector.

The modified antibodies of the present invention may be produced in eukaryotic cells such as CHO cells or human or murine hybridoma cells for example, as well as in transgenic plant and animal cells.

It is a further object of the present invention to provide prokaryotic or eukaryotic host cells, comprising a vector according to the invention.

Vectors suitable for expressing an anti-LF/EF antibody according to the invention are well known from the person skilled in the art, and may be an adenovirus, a retrovirus, a plasmid or a bacteriophage, this list being non limitative. In addition, any mammal cell may be used as the host cell, that is to say as a cell expressing nucleic acid fragments carried by the expression vector of the light chain of the IgG of the invention, for example YB2/0, CHO, CHO dhfr- (for example CHO DX B11, CHO DG44), CHO Lec13, SP2/0, NS0, 293, BHK or COS.

Thus, a vector of the invention may include the sequences encoding the heavy chain and/or the light chains of the immunoglobulin of the invention.

It is another object of the present invention to provide a host cell comprising the nucleic acids or the vector such as defined hereabove.

Advantageously, a host cell suitable for producing an anti-LF/EF antibody of the invention is selected from SP2/0, YB2/0, IR983F, Namalwa human myeloma, PERC6, CHO cell lines, in particular CHO-K-1, CHO-Lec10, CHO-Lec1, CHO-Lec13, CHO Pro-5, CHO dhfr-, Wil-2, Jurkat, Vero, Molt-4, COS-7, 293-HEK, BHK, K6H6, NS0, SP2/0-Ag 14 and P3X63Ag8.653.

In some embodiments, the anti-LF/EF antibody may be produced in YB2/0 rat hybridoma (YB2/3HL.P2.G11.16Ag.20 cell, filed in the American Type Culture Collection under accession number ATCC CRL-1662). This cell line was chosen because of its ability to produce antibodies having an ADCC activity (antibody-dependent cell-mediated cytotoxicity) that was improved as compared with antibodies of similar primary structure obtained for example in CHO cells

In one particular aspect of the present invention, the stable cell line expressing an anti-LF/EF antibody of the invention, and more particularly selected from the previously described group, did integrate one or both expression vector(s) of the heavy chain and of the light chain such as previously described.

It is a further object of the present invention to provide a composition comprising at least one recombinant antibody that is described in the present specification, and especially an anti-LF/EF antibody of the IgG isotype.

Especially for LF or EF toxins detection purposes, the recombinant antibody that is described in the present specification, and especially a recombinant antibody of the IgG isotype, may be labeled. Examples of suitable markers include enzymes, radioisotopes, fluorescent compounds, colloidal metals, chimioluminescent compounds, and bioluminescent compounds.

The binding methods of a marker to an antibody are well known from the person skilled in the art.

Another labeling method consists in coupling the antibody to low-molecular weight haptens, which haptens may be specifically modified through a second reaction. Examples of suitable haptens include biotin, which reacts with avidin, or dinitrophenol, pyridoxal or fluorescein, which may react with anti-hapten specific antibodies.

It is an object of the present invention to provide a LF or EF anthrax toxin detection kit. This kit comprises: a container comprising at least one recombinant antibody as described herein, which recombinant antibody may be labeled or not, optionally, a container comprising buffer solutions, and optionally a container comprising labeled IgG detecting means, such as a biotin-binding protein, for example avidin or streptavidine, bound to a reporter molecule, such as a fluorescent or enzymatic marker. This container may also comprise non-labeled IgG detecting means, i.e. substantially antibodies or antibody fragments.

Thus, a recombinant antibody of the invention may be used *in vitro,* for example in immunological assays wherein they are used in a liquid phase or immobilized on a solid-phase vehicle. Examples of well known vehicles include glass, polystyrene, polypropylene, polyethylene, dextran, nylon, amylase, natural or modified cellulose, polyacrylamide, agarose or magnetite. Examples of immunological assays using the anti-LF/EF antibody of the invention are radioimmunoassays, histoimmunological labeling techniques, ELISAs, Western blots, immunoprecipitation assays, immunodiffusion assays, complement fixation assays, Fluorescence-activated Cell Sorting assays (FACS) or protein-chip analyses.

It is a further object of the present invention to provide a method for detecting in vitro a LF-containing anthrax toxin or a EF-containing anthrax toxin, in a biological sample, comprising the steps of: contacting the sample with at least one recombinant antibody as described herein, and detecting the binding of the said antibody as an indication of the presence of a LF-containing anthrax toxin or an EF-containing anthrax toxin.

The biological sample may be liquid: for example saliva, urine, cerebrospinal fluid, serum or blood, or solid or semi-solid, for example tissues or feces or a solid tissue such as traditionally used in histological diagnosis.

It is a further object of the present invention to provide a method for detecting *in vivo* a LF-containing anthrax toxin or an EF-containing anthrax toxin, wherein a labeled anti-LF/EF antibody of the invention is administered to a subject. The administered amount of labeled anti-LF/EF antibody should be sufficient for the binding of the antibody to the toxin to be detected. The administered amount of labeled recombinant antibody will depend on some factors such as the age and sex of the subject, as well as on the stage of the disease. The administered amount may vary from 0.01 mg/kg to 50 mg/kg, preferably from 0.1 mg/kg to 20 mg/kg, and more preferably from 0.1 mg/kg to 2 mg/kg.

To perform the *in vivo* diagnosis, an anti-LF/EF antibody of the invention should be linked to a radioisotope, either directly or indirectly, through functional groups. Commonly used functional groups include for example diethylene-triamine-pentacetic acid (DTPA) and ethylene-diamine-tetraacetic acid (EDTA). Examples of radioisotope metal ions include ¹¹¹In, ⁹⁷Ru, ⁶⁷Ga, ⁶⁸Ga, ⁷²As, ⁸⁹Zr and ²⁰¹Tl.

An anti-LF/EF antibody of the invention may also be labeled with a paramagnetic isotope for a diagnosis using the magnetic resonance imaging (MRI) or through electron spin resonance (ESR). Positron-emitting gamma radioisotopes may also be used, such as ¹⁵⁷Gd, ⁵⁵Mn, ¹⁶²Dy, ⁶⁸Ga, ⁵²Cr, and ⁵⁶Fe

It is a further object of the present invention to provide a pharmaceutical composition comprising at least one recombinant antibody that is described in the present specification, and especially a anti-LF/EF antibody of the IgG isotype.

Said pharmaceutical composition preferably includes a pharmaceutically acceptable vehicle. Such a vehicle as used herein is intended to mean a non toxic material which does not interfere with the biological activity efficiency of the active components of the composition. The expression "pharmaceutically acceptable" does refer to a non toxic material which is compatible with a biological system such as a cell, a cell culture, a tissue or an organism. The vehicle characteristics will depend on the method of administration.

The present invention relates to the use of at least one recombinant antibody that is described in the present specification, and especially a recombinant antibody of the IgG isotype., for the preparation of a pharmaceutical composition or a medication for treating or preventing an infection with *Bacillus anthracis.*

As used herein, the term "prevention" means to prevent the occurrence of the disease in a subject, particularly a human, in whom the disease has not yet appeared.

As used herein, the term "treatment" corresponds to the inhibition of this disease, i.e. the discontinuation of its development, its regression, or the disappearance of the symptoms and consequences of the disease, or the disappearance of the causes of the disease.

The anti-LF/EF antibody of the invention may also be used *in vitro* or *in vivo* for monitoring the evolution of the treatment of the disease, for example by determining the increase or the decrease in the number of cells targeted by anthrax toxins or the change in the LF toxin concentration or in the EF toxin cncentration in a biological sample.

It is an object of the present invention to provide a method for treating a subject, preferably a human, potentially infected with *Bacillus anthracis,* wherein a therapeutically efficient amount of an anti-LF/EF antibody according to the invention is administered to said subject.

As used herein, the expression "therapeutically efficient amount" is intended to mean the amount which is sufficient for performing the treatment when administered to a subject which is in need of such a treatment. The therapeutic effective amount depends on the subject, on the stage of the disease to treat and on the method of administration, and may be determined through routine procedures by the person skilled in the art.

As used herein, the term "anthrax" is intended to mean any disease caused, either directly or indirectly, by an infection with *Bacillus anthracis.* Initial symptoms of an inhalational infection are similar to those of coryza (fever, muscular pain.). After a couple of days, these symptoms evolve towards serious problems of respiratory distress and septic shock. Inhalation of the bacteria anthrax is usually fatal.

Anthrax cutaneous infection occurs when the bacterium penetrates into the skin through a preexisting cutaneous interstice. This infection does initially cause the formation of a papule, which develops within two or three days to a vesicle, thereafter to a 1 to 3 cm-diameter ulceration with a central necrotic area. The anthrax gastrointestinal infection results from the consumption of contaminated meat and is characterized by an acute inflammation of the intestinal tract.

A therapeutically efficient amount corresponds to an amount that is sufficient for reducing the symptoms of the disease and the infection evolution. Such amount may vary depending on the age and sex of the subject and on the stage of the disease and will be determined by the person skilled in the art. A therapeutically efficient amount may vary from 0.01 mg/kg to 50 mg/kg, preferably from 0.1 mg/kg to 20 mg/kg, and more preferably from 0.1 mg/kg to 2 mg/kg, in one or several doses per day, for one day, or longer.

The administration method may be injection or gradual infusion. Injection may be of the intravenous, intraperitoneal, intramuscular, subcutaneous or transdermal type.

Preparations for parenteral administration may include sterile aqueous or non-aqueous solutions, suspensions or emulsions. Examples of non-aqueous solvents include propylene glycol, polyethylene glycol, vegetable oils, such as olive oil, or injectable organic esters, such as ethyl oleate. Aqueous vehicles include water, alcohol/water solutions, emulsions or suspensions.

Advantageously, the recombinant antibody of the invention is associated with a tetracycline-based prophylactic treatment. The recombinant antibody of the invention enables to shorten the tetracycline-based prophylaxis, by stopping, in a secured manner, after an exposure risk, through a tetracycline-based quick treatment ("short treatment") followed with an injection of the recombinant antibody of the invention.

Advantageously, the recombinant antibody of the invention is associated with a curative treatment with ciprofloxacin.

### Method for determining an epitope of interest

This invention further relates to a specifically designed method for determining an epitope of interest that is common to at least two target protein molecules, comprising the steps of:
a) providing a binding molecule substance, preferably an antibody or an antigen-binding fragment thereof, which binds to a first target protein molecule,
b) determining the occurrence of a cross-reactivity of the said substance with one or more further target protein molecules that are distinct from the said first target protein molecule, and going on the method if a cross-reactivity has been determined;
c) identifying (a) peptide region(s) exposed to the solvent on each of the target protein molecules, and going on the method when it is found at least one peptide region exposed to the solvent which is common to at least two of the said target protein molecules,
d) selecting the one or more common peptide region(s) identified at step c), as candidate epitopes of interest,
e) for each candidate epitope selected at step d), providing a collection of peptide variants where a stretch of 5 to 20 contiguous amino acid residues of the amino acid sequence of the candidate epitope, preferably a stretch of 10 contiguous amino acid residues of the amino acid sequence of the candidate epitope, is replaced by an alanine residue,
f) assay each of the peptide variants provided at step e) for its binding to the same binding molecule as that provided at step a), whereby, in a peptide variant that does not bind to the said binding molecule, the mutated stretch of 10 contiguous amino acid residues thereof is assigned as comprising a potential epitope of interest, which potential epitope of interest is selected at the end of the step,
g) for each potential epitopes of interest selected at step f), providing a collection of peptide variants where one amino acid residue is replaced by an alanine residue, so as to determine, in each potential epitope of interest, the individual amino acid residues that are involved in the binding with the binding molecule provided at step a), whereby the said individual amino acid residues of interest define the said epitope of interest.

The epitope mapping that is described above is illustrated in the examples herein. In this work, antigen residues were considered to be part of the epitope only if they contributed directly to antibody binding. Epitopes are generally composed of only a few such residues and they can be identified by mutation to alanine. This approach is based on the fact that interactions between antibodies and antigens depend on interactions between aminoacid side chains. The side chain of alanine is constituted of a methyl group thus it is very small, and substituting one of the key residues constituting an epitope with alanine weakens the interaction between the antigen and the antibody. Therefore, the involvement of a residue in an epitope may be tested by mutating it to alanine: a mutation weakening the affinity for the antibody shows that the residue is part of the epitope. For epitope mapping generally, the first step is for whole regions regarded as epitope candidates to be mutated to alanine (or "shaved to alanine"). In a second step, the residues constituting the regions previously tested positively are each individually mutated to alanine (or "scanned to alanine") to confirm and map precisely the epitope.

**Table 1 : sequences**

| **SEQ ID N°** | **Type** | **Description** |
|---|---|---|
| 1 | peptide | LF anthrax toxin |
| 2 | peptide | EF anthrax toxin |
| 3 | peptide | LF epitope |
| 4 | peptide | EF epitope |
| 5 | peptide | Consensus LF/EF epitope |
| 6 | peptide | VL region of the antibody |
| 7 | peptide | CL region of the antibody |
| 8 | peptide | Complete light chain of the antibody |
| 9 | peptide | VH region of the antibody |
| 10 | peptide | CH region of the antibody |
| 11 | peptide | Complete Heavy chain of the antibody |
| 12 | nucleic acid | VL region of the antibody |
| 13 | nucleic acid | CL region of the antibody |
| 14 | nucleic acid | Complete light chain of the antibody |
| 15 | nucleic acid | VH region of the antibody |
| 16 | nucleic acid | CH region of the antibody |
| 17 | nucleic acid | Complete Heavy chain of the antibody |

This invention is further illustrated, without in any way being limited to, the following examples

### EXAMPLES

### A. MATERIALS AND METHODS OF THE EXAMPLES

### A.1. Ethics Statement

All animal studies presented here were specifically approved by the Institut de Recherche Biomédicale des Armées ethics committee under authorization n° 2006/42.1, and were conducted according to European 2010/63/UE guidelines.

### A.2. ELISA and western blot

The cross-reactivity of scFv 2LF with EF was tested by ELISA. Briefly, microtiter plates were coated with 2 µg/ml of EF (List Biological Laboratories, Campbell, CA), while LF (List biological Laboratories) and KLH (Sigma-Aldrich, Saint-Quentin Fallavier, France) were utilized as positive and negative controls respectively. After blocking with PBS-BSA 3%, the plates were incubated with serial dilutions of scFv 2LF. The plates were then washed with PBS-Tween 0.1%, and bound scFv 2LF was detected using a monoclonal anti-polyhistidine antibody conjugated to peroxidase (A7058, Sigma-Aldrich). The reactivity of scFv 2LF with EF was further tested by western blotting under reducing conditions. Three samples of LF and EF were diluted (5, 2 and 1 µg/ml) in RunBlue LDS Sample Buffer (Expedeon, San Diego, USA) with _-mercaptoethanol, heated for 10 min at 80oC and separated by electrophoresis on a 12% SDS-PAGE gel using the Rapid Reducing Running Buffer (Expedeon). The samples were transferred to a polyvinylidene fluoride membrane (Immobilon-Psq, Millipore, Molsheim, France) using a Trans-Blot SD semidry electrophoretic transfer cell (Biorad, Marnes-la-Coquette, France). The membrane was blocked, then incubated with a scFv 2LF solution (50 µg/ml). Bound scFv 2LF was revealed with a monoclonal anti-polyhistidine antibody conjugated to peroxidase (A7058, Sigma-Aldrich).

### A.3. Affinity measurements

Affinity constants (KD) and dissociation constants (kd) of scFv 2LF and of the chimeric antibody encoded by the vector "HK622-41" for LF, EF and their variants were measured by surface plasmon resonance (SPR) with a Biacore X (Biacore, Uppsala, Sweden) instrument. ScFv 2LF was immobilized at a maximum of 250 resonance units on a CM5 chip (Biacore) via amine coupling. A flow rate of 30 µl/min was maintained during measurements. A minimum of seven dilutions of LF, EF and their variants (220 to 14 nM) in HBS-EP buffer (Biacore) was tested for 1500 s. After each dilution, the chip was regenerated with glycine 1.5 buffer (Biacore), run at 10 µl/min for 30 s. Results were analyzed with the Biaevaluation software (a 1:1 (Langmuir) binding model for KD measurements, and a 1:1 (Langmuir) dissociation model for kd measurements). If an LF variant did not interact with scFv 2LF, its presence was verified by a Biacore experiment using anti-LF polyclonal antibodies. For this, the variant was immobilized on a CM5 chip as described above, and serum drawn from the macaque hyper-immunized with LF was used for detection while the pre-immune serum was used as a control.

The affinity constants have been calculated by using the method described by Karlsson et al. (1991, J Immunol Methods, Vol. 145 : 229-240) and then checked by internal tests as described by Schuck et al. (1996, Abal. Biochem., Vol. 240 : 262-272).

### A.4. In vivo neutralization test

ScFv 2LF was reformatted as a full-sized IgG, IgG 2LF, by expression of its variable regions in fusion with human constant regions (Fc) of _1 and _ isotypes. Human constant regions were preferred to their murine counterparts because the IgG has therapeutic potential, and a human Fc ensures better tolerance. The *in vivo* neutralization test was adapted from a previous study [Kulshreshtha P, Bhatnagar R (2011) Inhibition of anthrax toxins with a bispecific monoclonal antibody that cross reacts with edema factor as well as lethal factor of Bacillus anthracis. Mol Immunol 48: 1958-1965]. The edema toxin (ET) was obtained by mixing equal weights of PA and EF. Twenty µg of ET, premixed with 0, 2, 5 or 10 µg of IgG 2LF in a final volume of 25 µl in PBS, or 25 µl of PBS alone as a control, were injected into the right rear footpad of five Balb/c mice. The sizes (dorsal/plantar) of the footpads were measured in triplicate, with digital vernier caliper.

### A.5. Identification of epitope candidates by combined identity and solvent exposure analysis

LF and EF sequences were retrieved under the access numbers P15917 and P40136, respectively. The ends of LFN and EFN were defined in accordance with sequence annotations (LFN begins at residue E27 and EFN begins at residue N64) and the two sequences were aligned with the CLUSTALW tool at the PBIL server (http://pbil.univ-lyon1.fr), using standard parameters (figure 4). For each position in LFN, a score of 1 or 0 was attributed when the EFN counterpart residue was identical or different, respectively. When alignment gaps occurred, a score of 0 was attributed to the LF residues having no counterparts in EF. A curve averaging the identity percentage over a window of five residues was calculated to visualize the portions shared between EFN and LFN, as a length of five residues was considered as corresponding to the length of epitopes [36]. X-ray crystallographic structures for LF (1J7N) [Pannifer AD, Wong TY, Schwarzenbacher R, Renatus M, Petosa C, et al. (2001) Crystal structure of the anthrax lethal factor. Nature 414: 229-233] and EF (1XFV) [37] were retrieved from the Protein Data Bank (www.rcsb.org). The solvent exposure for each residue on LFN and EFN structures was calculated using the ASAView tool [38], then averaged using a sliding window of five amino acids. The three curves representing these three averaged parameters (identity between LFN and EFN, solvent exposure of EF and solvent exposure of LF) are shown in figure 5. Epitope candidates were identified as regions where LFN and EFN identity, averaged on a sliding window of 5 residues, is superior to the mean identity between LFN and EFN, and where solvent exposure is high. The identity between LFN and EFN, the nature and the solvent exposure of each residue were studied individually to determine the ends of regions selected as epitope candidates. Once the epitope candidates were precisely defined, their solvent exposures were statistically tested (unpaired t test, GraphPad Prism 5, La Jolla, CA).

### A.6. Alanine shaving and scanning

Alanine shaving and scanning are two complementary methods, both consisting of directed mutagenesis of residues to alanine to reduce their side chains to methyl groups; this eliminates the interactions involving the side chains of these residues. Alanine shaving is the replacement of several contiguous amino acids in the antigen, and tests for the presence of an epitope in the corresponding region. When alanine shaving indicates that a region contains an epitope, each of the residues has to be individually mutated to alanine to localize the epitope precisely. It might be remarked that mutation to glycine, which has no side-chain beyond the _-carbon, introduces a conformational flexibility which may indirectly alter the epitope and the affinity, so that mutation to glycine is inappropriate for epitope mapping [Moreira IS, Fernandes PA, Ramos MJ (2007) Hot spots--a review of the proteinprotein interface determinant amino-acid residues. Proteins 68: 803-812]. Six genes, encoding LF and its variants, in which an epitope candidate had been shaved to alanine, were synthesized (Entelechon, Regensburg, Germany), inserted into the vector pET15B (Merck chemical, Darmstadt, Germany) and expressed as described below. The interaction between a variant and scFv 2LF was regarded as significantly altered if the kd was more than a three-fold lower than observed with the parental LF. For alanine scanning, each residue was individually mutated to alanine (Entelechon) and the corresponding variants were expressed as described below. If the mutation of a residue to alanine decreased the kd by more than a threefold, the mutated residue was regarded as being part of the epitope. All residues identified as composing the epitope were then simultaneously mutated to alanine, and the reactivity of thevariant was tested by Biacore. The epitope was confirmed if all immunoreactivity was lost. Expression and purification of LF, EF and their variants were as described for scFv 2LF [Pelat T, Hust M, Laffly E, Condemine F, Bottex C, et al. (2007) High-affinity, human antibody-like antibody fragment (single-chain variable fragment) neutralizing the], except that cytoplasmic extracts were obtained by sonication (1 min pulses of 6 seconds and 10 Watts, separated by pauses of 1 s) (Vibracell 7240S, Bioblock scientifique, Illkirch, France). A band corresponding to each variant was detected by SDS-PAGE in these cytoplasmic extracts, but not in those purified from non-transformed *Escherichia coli* (controls). Purity was estimated at 25% and was insufficient for precise measurement of concentrations, which in turn prevented the measurement of the affinity constant (KD). Since the dissociation constant (kd) correlates with binding affinity [Pellequer J-L, Van Regenmortel MHV (1993) Affinity of monoclonal antibodies to large multivalent antigens: influence of steric hindrance on antibody affinity constants calculated from Scatchard plots. MI 30: 955-958] and its measurement does not depend on concentration values, the present study was based on kd measurements.

### A.7. Identification of the epitope candidates by existing in silico methods

In addition to the *in silico* method developed here, 12 prediction methods available through web servers or stand-alone programs (8 predicting continuous epitopes, 4 predicting discontinuous epitope) were used to identify epitope candidates: BEPITOPE (continuous, Pellequer's method with TURNEE scale, default settings) [Odorico M, Pellequer J-L (2003) BEPITOPE: predicting the location of continuous epitopes and patterns in proteins. JMR 16: 20-22.], BEPITOPE (continuous, Pellequer's method with TURN33 scale, default settings, [39]), BepiPred (continuous, default settings,[ Larsen JE, Lund O, Nielsen M (2006) Improved method for predicting linear B-cell epitopes. Immunome Res 2: 2]), Ellipro (continuous, default settings, [Ponomarenko J, Bui HH, Li W, Fusseder N, Bourne PE, et al. (2008) ElliPro: a new structure-based tool for the prediction of antibody epitopes. BMC Bioinformatics 9: 514.]), LEP-LP (continuous, default settings, [Chang HT, Liu CH, Pai TW (2008) Estimation and extraction of B-cell linear epitopes predicted by mathematical morphology approaches. J Mol Recognit 21: 431-441]), BCPREDS (continuous, default settings, [El-Manzalawy Y, Dobbs D, Honavar V (2008) Predicting linear B-cell epitopes using string kernels. J Mol Recognit 21: 243-255]), Epitopia (continuous, default settings, [Rubinstein ND, Mayrose I, Martz E, Pupko T (2009) Epitopia: a web-server for predicting B-cell epitopes. BMC Bioinformatics 10: 287]), COBEpro (continuous, default settings, [Sweredoski MJ, Baldi P (2009) COBEpro: a novel system for predicting continuous B-cell epitopes. Protein Eng Des Sel 22: 113-120]), CBTOPE (discontinuous, default settings, [Ansari HR, Raghava GP (2010) Identification of conformational B-cell Epitopes in an antigen from its primary sequence. Immunome Res 6: 6]), Ellipro (discontinuous, default settings, [41]), DiscoTope (discontinuous, default settings, [47]), BEpro (discontinuous, default settings, select residues with score _ 0.5). A consensus epitope was defined as a region for which half of the methods predicted an epitope.

The present invention is further illustrated, without in any way being limited to, the following examples.

### Example 1 : Binding properties of an anti-anthrax toxin antibody

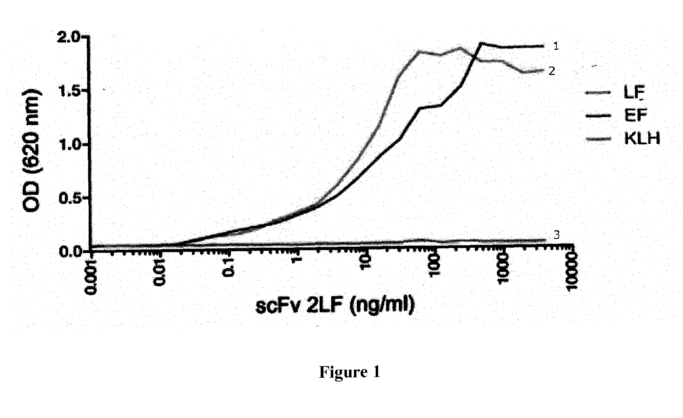

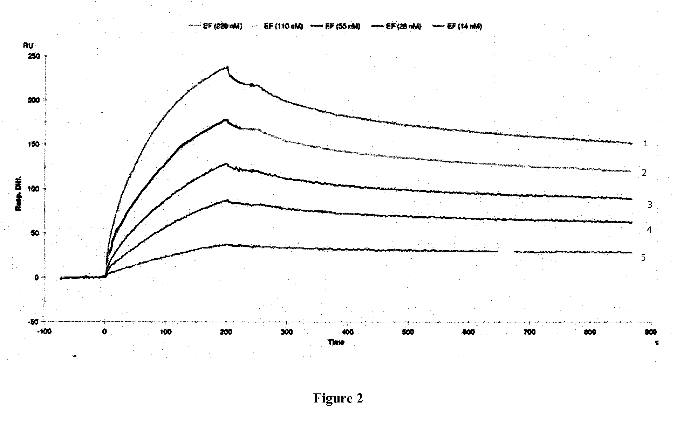

In ELISA, and in western blot under reducing conditions, scFv 2LF reacted with both EF and LF **(****figure 1**). The reactivity under reducing conditions indicated that the scFv 2LF epitopes on LF and EF are essentially linear. In a Biacore experiment, the affinity of scFv 2LF for EF was found to be 5 nM **(****figure 2****),** which is 5-fold lower than the affinity of scFv 2LF for LF (1.02 nM) [18]. This difference indicates that the epitopes ofscFv 2LF on EF and LF are not strictly identical.

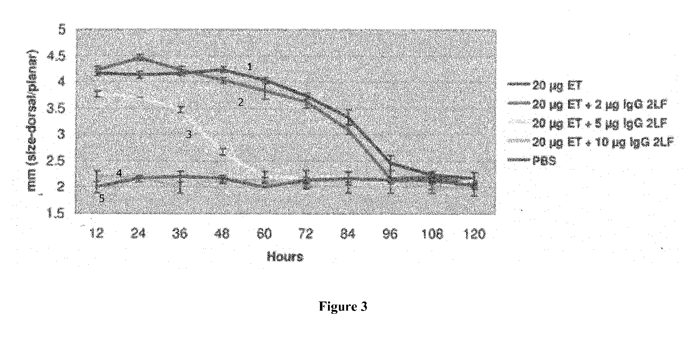

The capacity of scFv 2LF to neutralize ET was tested *in vivo.* Twenty µg of edema toxin (ET) injected into mouse footpads caused edema, culminating at the 12 hour after injection and returning to normal after 108 hours **(****figure 3****).** The edema was not reduced by premixing 2 µg of IgG-2LF with 20 µg ET. However, premixing 5 µg of IgG 2LF with 20 µg ET resulted in the edema being 25% smaller than that caused by ET alone, and the duration of edema (60 h vs. 108 h) being 45% shorter. Ten µg of IgG 2LF premixed with 20 µg ET completely inhibited edema formation **(****figure 3****),** and injection of PBS only did not induce edema. These data demonstrate that, in addition to neutralizing LT, scFv 2LF also neutralizes ET.

Further, using the affinity measurements disclosed in A.3. above, the respective affinity for LF as the value of the dissociation constant (KD) of the 2LF ScFv and the chimeric anti-LF/EF antibody encoded by the expression vector "HK622-41" have been determined. The affinity for LF of the 2LF ScFv has been measured as being 1;17 nM. The affinity for LF of the chimeric anti-LF/EF antibody encoded by the expression vector "HK622-41" has been measured as being 1.02 nM.

Consequently, it has been determined that the affinity for LF of the chimeric anti-LF/EF antibody encoded by the expression vector "HK622-41" is better than the affinity for LF of the 2LF ScFv.

### Example 2 : Determination of the epitopes recognized by the antibody both on LF anthrax toxin and on EF anthrax toxin

### Identification of epitope candidates by a new in silico method

ScFv 2LF blocks the interaction between LF and PA so that its epitope is presumably located in the N-terminal domain of LF, LFN [Pelat T, Hust M, Laffly E, Condemine F, Bottex C, et al. (2007) High-affinity, human antibody-like antibody fragment (single-chain variable fragment) neutralizing the lethal factor (LF) of Bacillus anthracis by inhibiting protective antigen-LF complex formation. Antimicrob Agents Chemother 51: 2758-2764]. Also, scFv 2LF cross-reacts with EF and LF, so its epitope is presumably highly conserved between the two proteins. To interact with an antibody, an epitope has indeed to be exposed to the solvent. Regions highly conserved between LFN and its homolog, the N-terminal domain of EF (EFN), were thus considered to be epitope candidates if they were exposed to the solvent. An *in silico* method was designed to search for such regions, and its main features were that (i) it was based on a sliding window of five residues, corresponding to the lengths of most epitopes, and residues constituting the extremities of candidate sequences were analyzed in detail (ii) the mean identity between the LFN and EFN sequences was calculated as equal to 35%, and this value was used as a threshold, above which regions were selected as candidates if they appeared to be exposed to the solvent (iii) the solvent exposure of candidates was verified statistically.

Starting from the N-terminus, the first region exposed to the solvent and conserved between LFN and EFN is located between positions 96 and 102, as it presents 56% identity. Residue 96 is an alanine, thus it is unlikely to participate significantly in the scFv 2LF binding and it was excluded from the candidate region. Position 103 was added as the corresponding residues in LF (K103) and EF (N103) are exposed to the solvent. The first epitope candidate was thus refined as LF(97-103) and its mean solvent exposure was 53% **(table 1).**

The second selected candidate region is located between residues 136 and 142, and its percentage identity is 57 %. Residue K143 was added to this candidate region as it is exposed on LF. The second candidate region retained for further testing was thus LF(136-143), with a mean solvent exposure of 47% **(table 1).**

The region between LF residues 180 and 184 shares 60% identity with EF, and is exposed to the solvent on both proteins. It was refined as LF(178-184), as residues K178 and N179 were added to compensate for the lack of homologs on EF. The mean solvent exposure of LF(178-184) is 52% **(table 1**).

The region located between 227 and 237 is very similar in LFN and EFN (73%), and exposed to the solvent. Because residue 237 is an alanine, only the region between residues 227 and 236 was retained for further study. It was divided into two regions, LF(227-231) and LF(231-236), to limit the number of residues needing to be tested in the following steps of the study. The mean solvent exposures of LF(227-231) and of LF(231-236) are 48% and 44%, respectively **(table 1).**

Five regions, LF(97-103), LF(136-143), LF(178-184), LF(227-231) and LF(231-236), were thus selected as epitope candidates and their mean solvent exposure is 48.1 %. The rest of the LFN and EFN regions sharing an identity greater than 35% had a mean solvent exposure of 19.7 %, which is significantly lower (p<0.0001) than the selected regions. The five selected LF regions were thus confirmed as epitope candidates ; they are constituted of 32 residues, thus far less than the 261 residues constituting LFN.

### Evaluation of the epitope candidates by alanine shaving.

If the mutation to alanine (or "shaving to alanine") of a region selected as an epitope candidate reduced the binding of the mutated LF to scFv 2LF, compared to the unmutated LF, the region was selected for subsequent analysis.

First, it was verified that the unmutated LF produced in our laboratory had a reactivity for scFv 2LF (kd= 2.3x10-4 s-1) equivalent to that of a commercially available LF (kd= 2.1x10-4 s-1). The residues of region LF(97-103) were substituted with alanine and the interaction between the mutated LF and scFv 2LF was tested. The observed kd was 1.32x10-4 s-1 **(****figure 6****),** thus equivalent to the value observed for the unmutated LF, so that this region was not studied further. Similarly, mutating the residues of region LF(136-143) to alanine led to a kd of 3x10-4 s-1 **(****figure 6****),** and therefore this second epitope candidate was also rejected. Substitution of the residues of LF(178-184) and of LF( 231-236) caused a complete loss of reactivity with scFv 2LF so that these regions were retained as candidates. Mutation of LF(227-231) to alanine reduced the reactivity with scFv 2LF to a kd of 1.26x10-3 s-1 ; this result suggested that LF(227-231) contributes to the interaction with scFv 2LF but that it is not essential for this interaction, and it was included in subsequent evaluations. Thus, alanine shaving identified three regions, LF(178- 184), LF(227-231) and LF(231-236), constituted of 17 residues, as candidates to be further studied by alanine scanning. These three regions are close to each other on the three-dimensional structure of LF.

### Precise identification of the epitope on LF and EF.

Each of the residues constituting LF(178-184), LF(227-231) and LF(231-236) were individually mutated to ("scanned to") alanine. The mutations of residues H229, R230, Q234, L235 and Y236 weakened the binding between LF and scFv 2LF, as the corresponding values of kd were at least 3-fold lower than that of the unmutated sequence **(Table 2).** Mutation to alanine of the other residues constituting these three regions did not substantially modify LF reactivity. The LF variant in which H229, R230, Q234, L235 and Y236 were all replaced with alanine did not bind scFv 2LF, demonstrating that the scFv 2LF epitope on LF is constituted by LF(229-230) plus LF(234-236). This epitope is linear as expected, but discontinuous.

We tested whether the homolog of this epitope in EF constitutes the epitope of scFv 2LF on EF. For this, a recombinant EF was produced in our laboratory and confirmed to present an reactivity with scFv 2LF similar to that of the commercially available EF (kd=5.1x10-4 s-1, **table 2).** The homologs of LF(229-230) and of LF(234-236) were identifed by sequence alignment as EF(253-254) and EF(258-260), respectively. These five residues were all mutated to alanine and the mutated EF was not recognized by scFv 2LF. The epitope of scFv 2LF on EF is thus EF(253-254) plus EF(258-260).

### Identification of the epitope candidates by existing in silico methods

Twelve *in silico* prediction methods, available through web servers or stand-alone programs, were used to map on LFN the epitope of scFv 2LF. To be in a consensus epitope, a residue must have been predicted as part of the epitope by at least six of the 12 methods. Twelve consensus epitopes were identified: LF(27-35), LF(49-55), LF(86-91), LF(98-104), LF(109-116), LF(124-125), LF(136-139), LF(164-169), LF(176-188), LF(191-210), LF(227-232) and LF(252-261), including a total of 98 residues. Although this consensus region was large, it did not include three (LF(234-236)) of the five residues demonstrated in the present study as constituting the epitope.

### DISCUSSION

An *in silico* analysis was used to search for epitope candidates, defined as LFN and EFN regions exposed to the solvent and presenting a high degree of identity. The degree of identity was averaged over a sliding window of five residues, a common size for an epitope, and regions with identity greater than the mean identity between LFN and EFN were selected. Five epitope candidates totaling 32 amino acids were thereby identified in the candidate domain, LFN, composed of 261 amino acids. This elimination of 87% of the residues greatly facilitated the subsequent experimental investigations, based on mutations to alanine.

The performance of the *in silico* method developed here was compared with that of 12 existing methods. The consensus epitope candidates (the epitope candidates indicated by at least half of these methods) consisted of 98 amino acids, thus was three times larger than the epitope candidates identified by our analysis. Four out of five epitope candidates (LF(97-103), LF(136- 143), LF(178-184), and LF(227-231)) identified in the present *in silico* study overlap with these consensus epitope candidates. Regarding the fifth epitope candidate identified by our analysis, LF(231-236), only residues 231 and 232 were among the consensus epitope candidates. Therefore, the consensus approach missed LF(234-236) though it constitutes three of the five residues of the epitope. Only four of the twelve preexisting methods indicated LF(234-236) as epitope candidate ; these comparisons highlight the usefulness of the *in silico* analysis presented here.

Here, alanine shaving was utilized to test the five epitope candidates selected by the *in silico* study. Shaving LF(178-184) and LF(231-236) completely abolished the interaction with scFv 2LF, and shaving LF(227-231) partially reduced the interaction. In these three regions, alanine scanning identified five residues involved in the interaction with scFv 2LF: H229, R230, Q234, L235 and Y236. The simultaneous mutation of these five amino acids to alanine completely abolished LF reactivity with scFv 2LF, demonstrating that they constitute the epitope of scFv 2LF on LF. This epitope partially corresponds to the _ helix 10, localized in the LF(231- 236) region. Alanine scanning showed that LF(178-184) has no direct role in the interaction between LF and scFv 2LF, even though this region was tested positively by alanine shaving. It is possible that LF(178-184) contributes to the conformation of _ helix 10, so that its role in scFv 2LF binding is indirect. This discordance between results obtained by alanine shaving and scanning examplify that alanine scanning is not only useful for precise epitope mapping, but also necessary to confirm the results of alanine shaving.

Three of the amino acids constituting the scFv 2LF epitope, Q234, L235 and Y236, are included in the LF(231-236) region, and alanine shaving of this region completely abolished the interaction with scFv 2LF. These three residues are therefore essential for the interaction. The two other residues, H229 and R230, are located within the LF(227-231) region, whose shaving only reduced the strength of the interaction, so that their role is likely to be less important.

The homolog on EF of the scFv 2LF epitope in LF was localized by sequence alignment as EF(253-254) and EF(258-260). The mutation to alanine of these two regions confirmed that they constitute the scFv 2LF epitope of EF. The residues H229, R230, L235 and Y236, part of the scFv 2LF epitope on LF, are strictly conserved in EF, respectively at positions 253, 254, 259 and 260. The glutamine residue at position 234 in LF corresponds to a glutamic acid residue at position 258 in EF; physicochemical properties of these two amino-acids differ only slightly but these differences presumably explain the 5-fold weaker interaction of scFv 2LF with EF than with LF.

If no epitope candidate had been tested positively by mutations to alanine, a second series of candidates would have been defined. For this, the mean exposure of residues constituting LFN (29%) would have been utilized as the solvent exposure threshold, in addition to the similarity threshold. These two thresholds, each defined as a mean value characterizing the antigens, would have identified five additional regions as epitope candidates, constituted of a total of 25 residues, and these regions would have been tested similarly than the first series.

Our first attempt to map the scFv 2LF epitope utilized five synthetic peptides corresponding to the candidates selected by the *in silico* analysis. Their reactivity with 2LF was tested by ELISA. The peptide corresponding to LF(97-103) was the only peptide that bound to the 2LF antibody, but it did not compete with LF for scFv 2LF binding (data not shown). For this reason, we used a different approach involving mutagenesis techniques guided by the same *in silico* results. As reported above, the mutagenesis experiments gave different results than the identification of LF(97-103) as epitope. This example illustrates how soluble peptides may present conformations different than those existing in the context of whole proteins, and consequently be misleading for epitope mapping. It also indicates that epitope mapping with peptides should be confirmed by competition experiments, which is not always the case in published studies.

In conclusion, we show in the present study that scFv 2LF cross-neutralizes the edema toxin and the lethal toxin, and we describe a novel *in silico* method based on this cross-reactivity to map its epitopes. This *in silico* method performed better than several existing methods, and may be of more general interest than the present study. The results of the *in silico* method were confirmed and refined by mutations to alanine (alanine shaving followed by alanine scanning), and these methods were found to be more robust than methods based on peptide utilization. The epitopes of scFv 2LF are constituted of LF(229-230) plus LF(234-236) in LF, and of EF(253- 254) plus EF(258-260) in EF. These epitopes are located in the region containing helix α10 and it may be possible to target this region with other inhibitors, such as synthetic molecules, or by vaccination, to neutralize both LT and ET in order to improve anthrax outcome.

**Table 1 : Mean identity between each epitope candidate in LF and the homologous region in EF, and mean solvent exposure of each epitope candidate.**

| The identities between EF and LF epitope candidates are higher than the threshold, equal to 35%. The calculated mean solvent exposure for the five epitope candidates is 48%. | | | |
|---|---|---|---|
| **Epitope candidate** | **length** | **Mean identity between the amino-acid sequence and the homologous region in EF (%)** | **Mean solvent exposure (%)** |
| LF(97-203) | 7 | 57 | 53 |
| LF(136-143) | 8 | 50 | 47 |
| LF(178-184) | 7 | 43 | 52 |
| LF(227-231) | 5 | 60 | 48 |
| LF(231-236) | 6 | 67 | 44 |

**Table 2 : ScFv 2LF dissociation rate constants with each LF variant, measured by Biacore experiments.**

| The five residues whose mutation to alanine resulted in a 3-fold, or larger, decrease of the dissociation constant are shown in bold. | | |
|---|---|---|
| | **residues** | **k_{d} (s⁻¹)** |
| **Shaving (SH)** | 97-103 | 1.32x10⁴ |
| | 136-143 | 3x10⁻⁴ |
| | 178-184 | / |
| | 227-23 | 1.26x10⁻³ |
| | 231-236 | / |
| **Scanning** | K178 | 2x10⁻⁴ |
| | N179 | 2.12x10⁻⁴ |
| | S181 | 3.1x10⁻⁴ |
| | D182 | 3.9x10⁻⁴ |
| | S183 | 2.9x10⁻⁴ |
| | D184 | 1.3x10⁻⁴ |
| | P227 | 5.6x10⁻⁴ |
| | Q228 | 6x10⁻⁴ |
| | **H229** | **10x10⁻⁴** |
| | **R230** | **9.34x10⁻⁴** |
| | D231 | 5.3x10⁻⁴ |
| | V232 | 5.24x10⁻⁴ |
| | L233 | 4.8x10⁻⁴ |
| | **Q234** | **9.54x10⁻⁴** |
| | **L235** | **8.95x10⁻⁴** |
| | **Y236** | **8.55x10⁻⁴** |
| **Native LF** | / | 2.3x10⁻⁴ |
| **Native EF** | / | 5.1x10⁻⁴ |

## Claims

1. A recombinant antibody comprising :
(i) a variable region binding to an epitope of the LF anthrax toxin comprising the amino acid residues H229, R230, Q234, L235 and Y236 thereof, and
(ii) a constant region of a human immunoglobulin of the gamma isotype

2. The recombinant antibody according to claim 1, wherein the variable region comprises :
- a VL region comprising an amino acid sequence having at least 90% amino acid identity with the amino acid sequence of SEQ ID N° 6, and
- a VH region comprising an amino acid sequence having at least 90% amino acid identity with the amino acid sequence of SEQ ID N° 9.

3. The recombinant antibody according to any one of claims 1 and 2, wherein the constant region is selected in the group comprising gamma 1, gamma 2 and gamma 3 isotype.

4. The recombinant antibody according to any one of claims 1 to 3, wherein the constant region comprises :
- a CL region comprising an amino acid sequence having at least 90% amino acid identity with the amino acid sequence of SEQ ID N° 7, and
- a CH region comprising an amino acid sequence having at least 90% amino acid identity with the amino acid sequence of SEQ ID N° 10.

5. A nucleic acid comprising nucleic acid sequences encoding a recombinant antibody according to any one of claims 1 to 4.

6. The nucleic acid according to claim 5, comprising one or more of the following nucleic acid sequences :
- a nucleic acid encoding the VL region, comprising a nucleic acid sequence having at least 90% nucleotide identity with SEQ ID N° 12,
- a nucleic acid encoding the VH region, comprising a nucleic acid sequence having at least 90% nucleotide identity with SEQ ID N° 15,
- a nucleic acid encoding the CL region, comprising a nucleic acid sequence having at least 90% nucleotide identity with SEQ ID N° 13, and
- a nucleic acid encoding the CH region, comprising a nucleic acid sequence having at least 90% nucleotide identity with SEQ ID N° 16.

7. A vector comprising a nucleic acid according to any one of claims 5 and 6.

8. A host cell comprising a vector according to claim 7.

9. A pharmaceutical composition comprising a recombinant antibody according to any one of claims 1 to 4, in combination with one or more pharmaceutically acceptable excipients.

10. The use of an antibody binding both to :
a) an epitope of the LF anthrax toxin comprising the amino acid residues H229, R230, Q234, L235 and Y236, and
b) an epitope of the EF anthrax toxin comprising the amino acid residues H253, R254, E258, L259 and Y260 thereof,
for preventing or treating a disorder or a disease caused by the presence of an anthrax toxin in an individual.

11. The use according to claim 10, wherein the said antibody is an antibody according to any one of claims 1 to 4.

12. A method for the screening of compounds that neutralize anthrax toxins, comprising the steps of:
a) bringing into contact a candidate compound with an LF anthrax toxin or a fragment thereof comprising the epitope comprising the amino acid residues H229, R230, Q234, L235 and Y236 thereof,
b) determining the occurrence of a binding event between the said candidate compound and the said LF anthrax toxin of the said fragment thereof,
c) selecting the said candidate compound if a binding event between the said candidate compound and the said LF anthrax toxin of the said fragment thereof is determined at step b).

13. The method according to claim 8, which further comprises the following steps :
d) bringing into contact a candidate compound with an EF anthrax toxin or a fragment thereof comprising the comprising the amino acid residues H253, R254, E258, L259 and Y260 thereof,
e) determining the occurrence of a binding event between the said candidate compound and the said EF anthrax toxin of the said fragment thereof,
f) selecting the said candidate compound if a binding event between the said candidate compound and the said EF anthrax toxin of the said fragment thereof is determined at step e).
and wherein the order of (i) steps a)-c) and (ii) steps d)-e), respectively, is indifferent.

14. The method according to any one of claims 12 and 13, wherein the candidate compound consists of an antibody or an antibody fragment comprising the variable region thereof.


SEQUENCE LISTING

<110>  Laboratoire Français du Fractionnement et des Biotechnologies

<120>  Antibody directed against anthrax toxins and its uses

<130>  PR97852

<160>  17

<170>  PatentIn version 3.5

<210>  1
<211>  809
<212>  PRT
<213>  Bacillus anthracis

<400>  1

Met Asn Ile Lys Lys Glu Phe Ile Lys Val Ile Ser Met Ser Cys Leu
1               5                   10                  15

Val Thr Ala Ile Thr Leu Ser Gly Pro Val Phe Ile Pro Leu Val Gln
            20                  25                  30

Gly Ala Gly Gly His Gly Asp Val Gly Met His Val Lys Glu Lys Glu
        35                  40                  45

Lys Asn Lys Asp Glu Asn Lys Arg Lys Asp Glu Glu Arg Asn Lys Thr
    50                  55                  60

Gln Glu Glu His Leu Lys Glu Ile Met Lys His Ile Val Lys Ile Glu
65                  70                  75                  80

Val Lys Gly Glu Glu Ala Val Lys Lys Glu Ala Ala Glu Lys Leu Leu
                85                  90                  95

Glu Lys Val Pro Ser Asp Val Leu Glu Met Tyr Lys Ala Ile Gly Gly
            100                 105                 110

Lys Ile Tyr Ile Val Asp Gly Asp Ile Thr Lys His Ile Ser Leu Glu
        115                 120                 125

Ala Leu Ser Glu Asp Lys Lys Lys Ile Lys Asp Ile Tyr Gly Lys Asp
    130                 135                 140

Ala Leu Leu His Glu His Tyr Val Tyr Ala Lys Glu Gly Tyr Glu Pro
145                 150                 155                 160

Val Leu Val Ile Gln Ser Ser Glu Asp Tyr Val Glu Asn Thr Glu Lys
                165                 170                 175

Ala Leu Asn Val Tyr Tyr Glu Ile Gly Lys Ile Leu Ser Arg Asp Ile
        180                 185                 190

Leu Ser Lys Ile Asn Gln Pro Tyr Gln Lys Phe Leu Asp Val Leu Asn
    195                 200                 205

Thr Ile Lys Asn Ala Ser Asp Ser Asp Gly Gln Asp Leu Leu Phe Thr
    210                 215                 220

Asn Gln Leu Lys Glu His Pro Thr Asp Phe Ser Val Glu Phe Leu Glu
225                 230                 235                 240

Gln Asn Ser Asn Glu Val Gln Glu Val Phe Ala Lys Ala Phe Ala Tyr
                245                 250                 255

Tyr Ile Glu Pro Gln His Arg Asp Val Leu Gln Leu Tyr Ala Pro Glu
            260                 265                 270

Ala Phe Asn Tyr Met Asp Lys Phe Asn Glu Gln Glu Ile Asn Leu Ser
        275                 280                 285

Leu Glu Glu Leu Lys Asp Gln Arg Met Leu Ala Arg Tyr Glu Lys Trp
    290                 295                 300

Glu Lys Ile Lys Gln His Tyr Gln His Trp Ser Asp Ser Leu Ser Glu
305                 310                 315                 320

Glu Gly Arg Gly Leu Leu Lys Lys Leu Gln Ile Pro Ile Glu Pro Lys
                325                 330                 335

Lys Asp Asp Ile Ile His Ser Leu Ser Gln Glu Glu Lys Glu Leu Leu
            340                 345                 350

Lys Arg Ile Gln Ile Asp Ser Ser Asp Phe Leu Ser Thr Glu Glu Lys
        355                 360                 365

Glu Phe Leu Lys Lys Leu Gln Ile Asp Ile Arg Asp Ser Leu Ser Glu
    370                 375                 380

Glu Glu Lys Glu Leu Leu Asn Arg Ile Gln Val Asp Ser Ser Asn Pro
385                 390                 395                 400

Leu Ser Glu Lys Glu Lys Glu Phe Leu Lys Lys Leu Lys Leu Asp Ile
                405                 410                 415

Gln Pro Tyr Asp Ile Asn Gln Arg Leu Gln Asp Thr Gly Gly Leu Ile
            420                 425                 430

Asp Ser Pro Ser Ile Asn Leu Asp Val Arg Lys Gln Tyr Lys Arg Asp
        435                 440                 445

Ile Gln Asn Ile Asp Ala Leu Leu His Gln Ser Ile Gly Ser Thr Leu
    450                 455                 460

Tyr Asn Lys Ile Tyr Leu Tyr Glu Asn Met Asn Ile Asn Asn Leu Thr
465                 470                 475                 480

Ala Thr Leu Gly Ala Asp Leu Val Asp Ser Thr Asp Asn Thr Lys Ile
                485                 490                 495

Asn Arg Gly Ile Phe Asn Glu Phe Lys Lys Asn Phe Lys Tyr Ser Ile
            500                 505                 510

Ser Ser Asn Tyr Met Ile Val Asp Ile Asn Glu Arg Pro Ala Leu Asp
        515                 520                 525

Asn Glu Arg Leu Lys Trp Arg Ile Gln Leu Ser Pro Asp Thr Arg Ala
    530                 535                 540

Gly Tyr Leu Glu Asn Gly Lys Leu Ile Leu Gln Arg Asn Ile Gly Leu
545                 550                 555                 560

Glu Ile Lys Asp Val Gln Ile Ile Lys Gln Ser Glu Lys Glu Tyr Ile
                565                 570                 575

Arg Ile Asp Ala Lys Val Val Pro Lys Ser Lys Ile Asp Thr Lys Ile
            580                 585                 590

Gln Glu Ala Gln Leu Asn Ile Asn Gln Glu Trp Asn Lys Ala Leu Gly
        595                 600                 605

Leu Pro Lys Tyr Thr Lys Leu Ile Thr Phe Asn Val His Asn Arg Tyr
    610                 615                 620

Ala Ser Asn Ile Val Glu Ser Ala Tyr Leu Ile Leu Asn Glu Trp Lys
625                 630                 635                 640

Asn Asn Ile Gln Ser Asp Leu Ile Lys Lys Val Thr Asn Tyr Leu Val
                645                 650                 655

Asp Gly Asn Gly Arg Phe Val Phe Thr Asp Ile Thr Leu Pro Asn Ile
            660                 665                 670

Ala Glu Gln Tyr Thr His Gln Asp Glu Ile Tyr Glu Gln Val His Ser
        675                 680                 685

Lys Gly Leu Tyr Val Pro Glu Ser Arg Ser Ile Leu Leu His Gly Pro
    690                 695                 700

Ser Lys Gly Val Glu Leu Arg Asn Asp Ser Glu Gly Phe Ile His Glu
705                 710                 715                 720

Phe Gly His Ala Val Asp Asp Tyr Ala Gly Tyr Leu Leu Asp Lys Asn
                725                 730                 735

Gln Ser Asp Leu Val Thr Asn Ser Lys Lys Phe Ile Asp Ile Phe Lys
            740                 745                 750

Glu Glu Gly Ser Asn Leu Thr Ser Tyr Gly Arg Thr Asn Glu Ala Glu
        755                 760                 765

Phe Phe Ala Glu Ala Phe Arg Leu Met His Ser Thr Asp His Ala Glu
    770                 775                 780

Arg Leu Lys Val Gln Lys Asn Ala Pro Lys Thr Phe Gln Phe Ile Asn
785                 790                 795                 800

Asp Gln Ile Lys Phe Ile Ile Asn Ser
                805

<210>  2
<211>  800
<212>  PRT
<213>  Bacillus anthracis

<400>  2

Met Thr Arg Asn Lys Phe Ile Pro Asn Lys Phe Ser Ile Ile Ser Phe
1               5                   10                  15

Ser Val Leu Leu Phe Ala Ile Ser Ser Ser Gln Ala Ile Glu Val Asn
            20                  25                  30

Ala Met Asn Glu His Tyr Thr Glu Ser Asp Ile Lys Arg Asn His Lys
        35                  40                  45

Thr Glu Lys Asn Lys Thr Glu Lys Glu Lys Phe Lys Asp Ser Ile Asn
    50                  55                  60

Asn Leu Val Lys Thr Glu Phe Thr Asn Glu Thr Leu Asp Lys Ile Gln
65                  70                  75                  80

Gln Thr Gln Asp Leu Leu Lys Lys Ile Pro Lys Asp Val Leu Glu Ile
                85                  90                  95

Tyr Ser Glu Leu Gly Gly Glu Ile Tyr Phe Thr Asp Ile Asp Leu Val
            100                 105                 110

Glu His Lys Glu Leu Gln Asp Leu Ser Glu Glu Glu Lys Asn Ser Met
        115                 120                 125

Asn Ser Arg Gly Glu Lys Val Pro Phe Ala Ser Arg Phe Val Phe Glu
    130                 135                 140

Lys Lys Arg Glu Thr Pro Lys Leu Ile Ile Asn Ile Lys Asp Tyr Ala
145                 150                 155                 160

Ile Asn Ser Glu Gln Ser Lys Glu Val Tyr Tyr Glu Ile Gly Lys Gly
                165                 170                 175

Ile Ser Leu Asp Ile Ile Ser Lys Asp Lys Ser Leu Asp Pro Glu Phe
            180                 185                 190

Leu Asn Leu Ile Lys Ser Leu Ser Asp Asp Ser Asp Ser Ser Asp Leu
        195                 200                 205

Leu Phe Ser Gln Lys Phe Lys Glu Lys Leu Glu Leu Asn Asn Lys Ser
    210                 215                 220

Ile Asp Ile Asn Phe Ile Lys Glu Asn Leu Thr Glu Phe Gln His Ala
225                 230                 235                 240

Phe Ser Leu Ala Phe Ser Tyr Tyr Phe Ala Pro Asp His Arg Thr Val
                245                 250                 255

Leu Glu Leu Tyr Ala Pro Asp Met Phe Glu Tyr Met Asn Lys Leu Glu
            260                 265                 270

Lys Gly Gly Phe Glu Lys Ile Ser Glu Ser Leu Lys Lys Glu Gly Val
        275                 280                 285

Glu Lys Asp Arg Ile Asp Val Leu Lys Gly Glu Lys Ala Leu Lys Ala
    290                 295                 300

Ser Gly Leu Val Pro Glu His Ala Asp Ala Phe Lys Lys Ile Ala Arg
305                 310                 315                 320

Glu Leu Asn Thr Tyr Ile Leu Phe Arg Pro Val Asn Lys Leu Ala Thr
                325                 330                 335

Asn Leu Ile Lys Ser Gly Val Ala Thr Lys Gly Leu Asn Val His Gly

340                 345                 350

Lys Ser Ser Asp Trp Gly Pro Val Ala Gly Tyr Ile Pro Phe Asp Gln
        355                 360                 365

Asp Leu Ser Lys Lys His Gly Gln Gln Leu Ala Val Glu Lys Gly Asn
    370                 375                 380

Leu Glu Asn Lys Lys Ser Ile Thr Glu His Glu Gly Glu Ile Gly Lys
385                 390                 395                 400

Ile Pro Leu Lys Leu Asp His Leu Arg Ile Glu Glu Leu Lys Glu Asn
                405                 410                 415

Gly Ile Ile Leu Lys Gly Lys Lys Glu Ile Asp Asn Gly Lys Lys Tyr
            420                 425                 430

Tyr Leu Leu Glu Ser Asn Asn Gln Val Tyr Glu Phe Arg Ile Ser Asp
        435                 440                 445

Glu Asn Asn Glu Val Gln Tyr Lys Thr Lys Glu Gly Lys Ile Thr Val
    450                 455                 460

Leu Gly Glu Lys Phe Asn Trp Arg Asn Ile Glu Val Met Ala Lys Asn
465                 470                 475                 480

Val Glu Gly Val Leu Lys Pro Leu Thr Ala Asp Tyr Asp Leu Phe Ala
                485                 490                 495

Leu Ala Pro Ser Leu Thr Glu Ile Lys Lys Gln Ile Pro Gln Lys Glu
            500                 505                 510

Trp Asp Lys Val Val Asn Thr Pro Asn Ser Leu Glu Lys Gln Lys Gly
        515                 520                 525

Val Thr Asn Leu Leu Ile Lys Tyr Gly Ile Glu Arg Lys Pro Asp Ser
    530                 535                 540

Thr Lys Gly Thr Leu Ser Asn Trp Gln Lys Gln Met Leu Asp Arg Leu
545                 550                 555                 560

Asn Glu Ala Val Lys Tyr Thr Gly Tyr Thr Gly Gly Asp Val Val Asn
                565                 570                 575

His Gly Thr Glu Gln Asp Asn Glu Glu Phe Pro Glu Lys Asp Asn Glu
            580                 585                 590

Ile Phe Ile Ile Asn Pro Glu Gly Glu Phe Ile Leu Thr Lys Asn Trp
        595                 600                 605

Glu Met Thr Gly Arg Phe Ile Glu Lys Asn Ile Thr Gly Lys Asp Tyr
    610                 615                 620

Leu Tyr Tyr Phe Asn Arg Ser Tyr Asn Lys Ile Ala Pro Gly Asn Lys
625                 630                 635                 640

Ala Tyr Ile Glu Trp Thr Asp Pro Ile Thr Lys Ala Lys Ile Asn Thr
                645                 650                 655

Ile Pro Thr Ser Ala Glu Phe Ile Lys Asn Leu Ser Ser Ile Arg Arg
            660                 665                 670

Ser Ser Asn Val Gly Val Tyr Lys Asp Ser Gly Asp Lys Asp Glu Phe
        675                 680                 685

Ala Lys Lys Glu Ser Val Lys Lys Ile Ala Gly Tyr Leu Ser Asp Tyr
    690                 695                 700

Tyr Asn Ser Ala Asn His Ile Phe Ser Gln Glu Lys Lys Arg Lys Ile
705                 710                 715                 720

Ser Ile Phe Arg Gly Ile Gln Ala Tyr Asn Glu Ile Glu Asn Val Leu
                725                 730                 735

Lys Ser Lys Gln Ile Ala Pro Glu Tyr Lys Asn Tyr Phe Gln Tyr Leu
            740                 745                 750

Lys Glu Arg Ile Thr Asn Gln Val Gln Leu Leu Leu Thr His Gln Lys
        755                 760                 765

Ser Asn Ile Glu Phe Lys Leu Leu Tyr Lys Gln Leu Asn Phe Thr Glu
    770                 775                 780

Asn Glu Thr Asp Asn Phe Glu Val Phe Gln Lys Ile Ile Asp Glu Lys
785                 790                 795                 800

<210>  3
<211>  8
<212>  PRT
<213>  artificial sequence

<220>
<223>  epitope

<400>  3

His Arg Asp Val Leu Gln Leu Tyr

1               5


<210>  4
<211>  8
<212>  PRT
<213>  artificial sequence

<220>
<223>  epitope

<400>  4

His Arg Thr Val Leu Glu Leu Tyr
1               5


<210>  5
<211>  8
<212>  PRT
<213>  artificial sequence

<220>
<223>  epitope


<220>
<221>  VARIANT
<222>  (3)..(3)
<223>  X means D or T

<220>
<221>  VARIANT
<222>  (6)..(6)
<223>  X means Q or E

<400>  5

His Arg Xaa Val Leu Xaa Leu Tyr
1               5


<210>  6
<211>  107
<212>  PRT
<213>  artificial sequence

<220>
<223>  VL region of antibody

<400>  6

Asp Ile Gln Met Thr Gln Ser Pro Ser Ser Val Ser Ala Ser Val Gly
1               5                   10                  15


Asp Arg Val Thr Ile Thr Cys Arg Ala Ser Gln Gly Ile Ser Ser Trp
            20                  25                  30


Leu Ala Trp Tyr Gln Gln Lys Pro Gly Lys Ala Pro Lys Leu Leu Ile
        35                  40                  45

Tyr Lys Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Ser Pro Ile
                85                  90                  95

Thr Phe Gly Gly Gly Thr Lys Val Glu Ile Lys
            100                 105

<210>  7
<211>  107
<212>  PRT
<213>  artificial sequence

<220>
<223>  CL region of antibody

<400>  7

<210>  8
<211>  214
<212>  PRT
<213>  artificial sequence

<220>
<223>  Complete heavy chain of antibody

<400>  8

Asp Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Ser Pro Ile
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  9
<211>  122
<212>  PRT
<213>  artificial sequence

<220>
<223>  VH region of antibody

<400>  9

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Ala Phe Asp Ser Thr
            20                  25                  30

Ala Met His Trp Val Arg Gln Ala Pro Gly Lys Gly Leu Glu Trp Val
        35                  40                  45

Ala Val Ile Ser Thr Gly Gly Ser Thr Tyr Tyr Ala Asp Ser Val Lys
    50                  55                  60

Gly Arg Phe Thr Ile Ser Arg Asp Asn Ser Asn Asn Thr Leu Tyr Leu
65                  70                  75                  80

Gln Met Asn Ser Leu Arg Ala Glu Asp Thr Ala Val Tyr Tyr Cys Ala
                85                  90                  95

Lys Ala Val Arg Gly Tyr Ile Gly Ser Arg Gly Pro Leu Asp Val Trp
            100                 105                 110

Gly Gln Gly Thr Met Val Thr Val Ser Ser
        115                 120


<210>  10
<211>  330
<212>  PRT
<213>  artificial sequence

<220>
<223>  CH region of antibody

<400>  10

<210>  11
<211>  452
<212>  PRT
<213>  artificial sequence

<220>
<223>  Complete heavy chain of antibody

<400>  11

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Ala Phe Asp Ser Thr
            20                  25                  30

Ala Met His Trp Val Arg Gln Ala Pro Gly Lys Gly Leu Glu Trp Val
        35                  40                  45

Ala Val Ile Ser Thr Gly Gly Ser Thr Tyr Tyr Ala Asp Ser Val Lys
    50                  55                  60

Gly Arg Phe Thr Ile Ser Arg Asp Asn Ser Asn Asn Thr Leu Tyr Leu
65                  70                  75                  80

Gln Met Asn Ser Leu Arg Ala Glu Asp Thr Ala Val Tyr Tyr Cys Ala
                85                  90                  95

Lys Ala Val Arg Gly Tyr Ile Gly Ser Arg Gly Pro Leu Asp Val Trp
            100                 105                 110

Gly Gln Gly Thr Met Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys
    450

<210>  12
<211>  387
<212>  DNA
<213>  artificial sequence

<220>
<223>  nucleic acid encoding VL region of antibody

<400>  12
atggacatga gggtgccagc tcagctgctg ggactgctgc tgctgtggct gccaggcgcc      60

aggtgcgaca tccagatgac ccagagccca agcagcgtga gcgccagcgt gggcgacagg     120

gtgaccatca cctgcagggc cagccagggc atcagcagct ggctggcctg gtaccagcag     180

aagccaggca aggctcccaa gctgctgatc tacaaggcca gcagcctgca gagcggagtg     240

ccaagcaggt tcagcggaag cggaagcgga accgacttca ccctgaccat cagcagcctg     300

cagcccgagg acttcgccac ctactactgc cagcagtaca gcaccagccc catcaccttc     360

ggtggcggaa ccaaggtgga gatcaag                                         387

<210>  13
<211>  327
<212>  DNA
<213>  artificial sequence

<220>
<223>  nucleic acid encoding CL region of antibody

<400>  13
aggaccgtgg ccgcaccaag tgtcttcatc tttcctccga gtgatgagca gctgaagagc      60

gggacagctt ctgtggtgtg tctgctgaat aacttctacc caagagaagc aaaggtccag     120

tggaaggtgg acaacgccct gcagtctggc aactcacagg agtctgtcac tgagcaggat     180

tccaaggaca gcacttacag cctgtccagc accctcactc tgtccaaagc cgactacgaa     240

aagcataagg tgtatgcttg tgaggtgacc caccagggac tgagcagccc tgtgacgaag     300

tccttcaacc ggggcgagtg ctagtga                                         327

<210>  14
<211>  714
<212>  DNA
<213>  artificial sequence

<220>
<223>  nucleic acid encoding complete light chain of antibody

<400>  14
atggacatga gggtgccagc tcagctgctg ggactgctgc tgctgtggct gccaggcgcc      60

aggtgcgaca tccagatgac ccagagccca agcagcgtga gcgccagcgt gggcgacagg     120

gtgaccatca cctgcagggc cagccagggc atcagcagct ggctggcctg gtaccagcag 180

aagccaggca aggctcccaa gctgctgatc tacaaggcca gcagcctgca gagcggagtg 240

ccaagcaggt tcagcggaag cggaagcgga accgacttca ccctgaccat cagcagcctg 300

cagcccgagg acttcgccac ctactactgc cagcagtaca gcaccagccc catcaccttc 360

ggtggcggaa ccaaggtgga gatcaagagg accgtggccg caccaagtgt cttcatcttt 420

cctccgagtg atgagcagct gaagagcggg acagcttctg tggtgtgtct gctgaataac 480

ttctacccaa gagaagcaaa ggtccagtgg aaggtggaca acgccctgca gtctggcaac 540

tcacaggagt ctgtcactga gcaggattcc aaggacagca cttacagcct gtccagcacc 600

ctcactctgt ccaaagccga ctacgaaaag cataaggtgt atgcttgtga ggtgacccac 660

cagggactga gcagccctgt gacgaagtcc ttcaaccggg gcgagtgcta gtga 714

<210> 15
<211> 423
<212> DNA
<213> artificial sequence

<220>
<223> nucleic acid encoding VH region of antibody

<400> 15
atgaagcacc tgtggttctt cctgctgctg gtggctgccc ccaggtgggt gctgagccag 60

gtgcagctgg tggagagcgg tggcggagtg gtgcagccag gcaggagcct gaggctgagc 120

tgcgctgcca gcggattcgc cttcgacagc accgctatgc attgggtgag gcaggctcca 180

ggcaagggcc tggagtgggt ggccgtgatc agcaccggcg gcagcaccta ctacgccgac 240

agcgtgaagg gcaggttcac catcagcagg gacaacagca acaacaccct gtacctgcag 300

atgaacagcc tgagggccga ggacaccgcc gtgtactact gcgctaaggc cgtgagggga 360

tacatcggaa gcaggggacc tctggacgtg tggggacagg gaaccatggt gaccgtgagc 420

agc 423

<210> 16
<211> 996
<212> DNA
<213> artificial sequence

<220>
<223> nucleic acid region encoding CH region of antibody

<400> 16
gccagcacca agggcccatc cgtgttcccc ctggccccat ccagcaagtc tacctccgga 60

ggcacagccg ccctgggctg tctggtgaag gactacttcc ccgagccagt gaccgtgtcc 120

tggaactccg gagccctgac atccggcgtg cacaccttcc ccgccgtgct gcagtccagc 180

ggcctgtact ctctgtcttc cgtggtgacc gtgccatcca gctccctggg aacccagaca 240

tacatctgca acgtgaacca caagcctagc aacaccaagg tggacaagaa ggtggagcct 300

aagagctgtg acaagacaca cacatgccct ccttgtccag cccctgagct gctgggcggc 360

ccctccgtgt tcctgttccc ccccaagcct aaggataccc tgatgatcag cagaaccccc 420

gaggtgacct gcgtggtggt ggacgtgtcc cacgaggatc ccgaggtgaa gttcaactgg 480

tacgtggacg gcgtggaggt gcacaacgct aagaccaagc ccagagagga gcagtacaac 540

agcacataca gagtggtgtc tgtgctgacc gtgctgcacc aggactggct gaacgggaag 600

gagtacaagt gcaaggtgtc caacaaggcc ctgcctgccc ctatcgagaa gaccatctct 660

aaggctaagg ggcagccccg ggagccacag gtgtacaccc tgccacccag ccgcgacgag 720

ctgaccaaga accaggtgtc cctgacatgc ctggtgaagg gattctaccc cagcgacatc 780

gccgtggagt gggagagcaa cggccagccc gagaacaact acaagacaac ccctcccgtg 840

ctggacagcg atggatcctt cttcctgtac tccaagctga ccgtggacaa gagcaggtgg 900

cagcagggaa acgtgttctc ttgttccgtg atgcacgagg ctctgcacaa ccactacacc 960

cagaagtccc tgagcctgtc tccaggcaag tgatag 996

<210> 17
<211> 1419
<212> DNA
<213> artificial sequence

<220>
<223> nucleic acid encoding complete heavy chain of antibody

<400> 17
atgaagcacc tgtggttctt cctgctgctg gtggctgccc ccaggtgggt gctgagccag 60

gtgcagctgg tggagagcgg tggcggagtg gtgcagccag gcaggagcct gaggctgagc 120

tgcgctgcca gcggattcgc cttcgacagc accgctatgc attgggtgag gcaggctcca 180

ggcaagggcc tggagtgggt ggccgtgatc agcaccggcg gcagcaccta ctacgccgac 240

agcgtgaagg gcaggttcac catcagcagg gacaacagca acaacaccct gtacctgcag 300

atgaacagcc tgagggccga ggacaccgcc gtgtactact gcgctaaggc cgtgagggga 360

tacatcggaa gcaggggacc tctggacgtg tggggacagg gaaccatggt gaccgtgagc 420

agcgccagca ccaagggccc atccgtgttc cccctggccc catccagcaa gtctacctcc 480

ggaggcacag ccgccctggg ctgtctggtg aaggactact tccccgagcc agtgaccgtg 540

tcctggaact ccggagccct gacatccggc gtgcacacct tccccgccgt gctgcagtcc 600

agcggcctgt actctctgtc ttccgtggtg accgtgccat ccagctccct gggaacccag 660

acatacatct gcaacgtgaa ccacaagcct agcaacacca aggtggacaa gaaggtggag 720

cctaagagct gtgacaagac acacacatgc cctccttgtc cagcccctga gctgctgggc 780

ggcccctccg tgttcctgtt cccccccaag cctaaggata ccctgatgat cagcagaacc 840

cccgaggtga cctgcgtggt ggtggacgtg tcccacgagg atcccgaggt gaagttcaac 900

tggtacgtgg acggcgtgga ggtgcacaac gctaagacca agcccagaga ggagcagtac 960

aacagcacat acagagtggt gtctgtgctg accgtgctgc accaggactg gctgaacggg 1020

aaggagtaca agtgcaaggt gtccaacaag gccctgcctg cccctatcga gaagaccatc 1080

tctaaggcta aggggcagcc ccgggagcca caggtgtaca ccctgccacc cagccgcgac 1140

gagctgacca agaaccaggt gtccctgaca tgcctggtga agggattcta ccccagcgac 1200

atcgccgtgg agtgggagag caacggccag cccgagaaca actacaagac aacccctccc 1260

gtgctggaca gcgatggatc cttcttcctg tactccaagc tgaccgtgga caagagcagg 1320

tggcagcagg gaaacgtgtt ctcttgttcc gtgatgcacg aggctctgca caaccactac 1380

acccagaagt ccctgagcct gtctccaggc aagtgatag 1419